# EUROPEAN PATENT SPECIFICATION

(11) **EP 1 648 868 B1**
(45) Date of publication and mention of the grant of the patent: **15.11.2006**
(21) Application number: 04742999.8
(22) Date of filing: 18.06.2004
(51) Int. Cl.: C07D 211/96, A61K 31/451, A61P 37/00

(54) **HYDROXAMATE SULFONAMIDES AS CD23 SHEDDING INHIBITORS**
HYDROXAMATSULFONAMIDE ALS INHIBITOREN VON CD23-SHEDDING
HYDROXAMATE SULFONAMIDES UTILISES EN TANT QU'INHIBITEURS DE L'ELIMINATION DE CD23

(30) Priority: 19.06.2003 GB 0314246; 05.11.2003 GB 0325832
(43) Date of publication of application: 26.04.2006
(73) Proprietor: Celltech R & D Limited, Slough, Berkshire SL1 3WE (GB)
(72) Inventor: OWEN, David Alan, CELLTECH R & D LIMITED, Slough Berkshire SL1 3WE (GB); WATSON, Robert John, CELLTECH R & D LIMITED, Slough Berkshire SL1 3WE (GB); ALLEN, Daniel Rees, CELLTECH R & D LIMITED, Slough Berkshire SL1 3WE (GB); SHARPE, Andrew, CELLTECH R & D LIMITED, Slough Berkshire SL1 3WE (GB); DYKE, Hazel Joan, CELLTECH R & D LIMITED, Slough Berkshire SL1 3WE (GB)
(74) Representative: Thompson, John
(86) International application number: PCT/GB2004/002646
(87) International publication number: WO 2004/113298

(56) References cited:
- WO-A-00/12477
- WO-A-01/62715
- WO-A-01/85721
- MAYER R J ET AL: "CD23 shedding: requirements for substrate recognition and inhibition by dipeptide hydroxamic acids" INFLAMMATION RESEARCH, vol. 51, 2002, pages 085-090, XP002296705

## Description

### Field of the Invention

This invention relates to a series of novel hydroxamate sulfonamides and their derivatives, to processes for their preparation, to pharmaceutical compositions containing them and to their use in medicine.

### Background of the Invention

CD23, which is also known as the low affinity receptor for immunoglobulin (Ig)E (FcεRII), is a type II integral protein expressed on a variety of haematopoietic and structural cells. In humans CD23 is a Ca²⁺ dependent C-type lectin of 45 kDa and exists under two forms, CD23a and CD23b (*Clin. and Exp. Allergy,* 2000, **30**, pp. 602-605). Both types are found on B-cells; CD23a is expressed constitutively and CD23b is induced in particular by IL-4. The b isoform is also found on non-B-cells such as T-cells, Langerhans cells, monocytes, macrophages, platelets and eosinophils.

CD23 is not only an IgE receptor, but also a membrane-bound precursor of soluble molecules that still bind IgE (sCD23 or IgE-binding factors) (Sarfati, M. *et al., Immunol. Res.,* 1992, **11**, pp. 260-272). sCD23 of molecular weights 37, 33, 29, 25 and 17 kDa arise by an autocatalytic cleavage process involving a metalloprotease cleavage of membrane-bound CD23 (Marolewski, A. *et al., Biochem. J.,* 1998, **333**, pp. 573-579).

Membrane-bound CD23 is a multifunctional molecule, which may exert different functions according to the cell type on which it is expressed, ranging from cellular adhesion, antigen presentation, growth and differentiation of B- and T-cells, rescue from apoptosis, release of cytotoxic mediators and regulation of IgE synthesis (Bonnefoy, J. *et al., Int. Rev. Immunol.,* 1997, **16**, pp. 113-128). It has been postulated that CD23 is overexpressed in several pathologic conditions such as allergic, autoimmune, and parasite diseases, and B-cell lymphoproliferative diseases, such as chronic lymphocytic leukemia.

There is increasing evidence that sCD23 fragments may exert several effects, either alone or in conjunction with other cytokines, on a large variety of haematopoietic cells. These effects include the regulation of IgE synthesis, promotion of B- and T-cell proliferation, and inhibition of monocyte migration, and in synergy with interleukin 1 (IL1) sCD23 fragments may be implicated in the differentiation of early thymocytes, myeloid cell precursors and some germinal centre B-cells.

In particular the three higher molecular weight sCD23 fragments (37, 33 and 29 kDa) have multifunctional cytokine properties which appear to play a major role in IgE production. The excessive formation of sCD23 has been implicated in the overproduction of IgE, which is the hallmark of allergic diseases such as extrinsic asthma, rhinitis, allergic conjunctivitis, eczema, atopic dermatitis and anaphylaxis (Sutton and Gould, *Nature,* 1993, **366**, pp. 421-428). Elevated levels of sCD23 have also been observed in the synovial fluids of patients with rheumatoid arthritis (Chomarat, P. *et al., Arthritis and Rheumatism,* 1993, **36**, pp. 234-242).

It has been shown that crosslinking CD23 at the cell surface by IgE delivers a negative feedback for IgE production and inhibits the release of sCD23. However, sCD23 fragments larger than 25 kDa that retain part of the stalk region may promote IgE production by at least two mechanisms: 1) sCD23 directly stimulates IgE production possibly through CD21 triggering; 2) sCD23 fragments are capable of trapping IgE in the medium and thus may prevent negative feedback through membrane-bound CD23. Thus, compounds which have the ability to inhibit the formation of sCD23 should have twofold actions of: 1) inhibiting the immunostimulatory activities of the higher molecular weight soluble fragments; 2) enhancing negative feedback inhibition of IgE synthesis by maintaining levels of CD23 on the surface of B-cells. In addition, inhibition of CD23 cleavage should lessen sCD23-induced monocyte activation and mediator formation, thereby reducing the inflammatory response.

Until recently the therapeutic approach to modulating allergic responses has been focussed on the mediators thought to cause the response rather than addressing directly the control of IgE production (Christie, G. *et al., Eur. J. Immunol.,* 1997, **27,** pp. 3228-3235). One proposed approach for a therapeutically relevant control point in the regulation of IgE synthesis is the regulation of CD23 processing to sCD23. CD23 inhibitors are disclosed in WO 0185721, WO 0162715 and Inflamm. Res. 51, 2002, 85-90. WO 0012477 discloses hydroxamic acid derivatives as proteinase inhibitors.

### Summary of the Invention

We have now found a class of hydroxamate sulfonamides which are potent inhibitors of CD23 shedding. Therefore the compounds are particularly suitable for the treatment and/or prophylaxis of allergic diseases associated with IgE production.

Thus we provide a compound of formula (1): 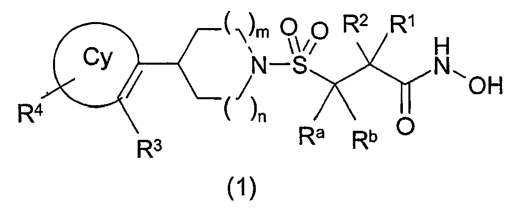 wherein:
Cy is an aryl or heteroaryl group;
m is zero or the integer 1, 2 or 3;
n is zero or the integer 1, 2 or 3; in which the sum of m and n is zero or the integer 1, 2 or 3;
R¹ is a group selected from C₁₋₆alkyl, aryl, heteroaryl, heterocycloalkyl, C₃₋₆cycloalkyl, -C₁₋₆alkylaryl, -C₁₋₆alkylheteroaryl, -C₁₋₆alkylheterocycloalkyl or -C₁₋₆alkylC₃₋₆cycloalkyl, in which each aryl or heteroaryl group, present as or as part of the group R¹, may optionally be substituted with 1, 2 or 3 substituents selected from the group R⁷, wherein each R⁷ may be the same or different, and is an atom or group selected from F, Cl, Br, C₁₋₆alkyl, C₁₋₆haloalkyl, C₁₋₆alkoxy, C₁₋₆haloalkoxy, -CN, -CO₂R^{7a}, -CON(R^{7a})₂ or -COR^{7a}; and in which each alkyl, heterocycloalkyl or cycloalkyl group, present as or as part of the group R¹, may optionally be substituted with 1, 2 or 3 substituents selected from the group R⁸, wherein each R⁸ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R^{7a}, which may be the same or different, is each a hydrogen atom, or a C₁₋₆alkyl or C₁₋₆haloalkyl group;
R^{8a}, which may be the same or different, is each a hydrogen atom, or a C₁₋₆alkyl or C₁₋₆haloalkyl group;
R¹⁰ is a hydrogen atom or a C₁₋₃alkyl group;
R² is a hydrogen atom or a C₁₋₃alkyl group;
or R¹ and R² together with the carbon atom to which they are attached form a C₃₋₆cycloalkyl or heterocycloalkyl group optionally substituted with 1, 2 or 3 substituents selected from the group R⁹, wherein each R⁹ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R³ is an atom or group selected from F, Cl, Br, C₁₋₃alkyl, C₁₋₃haloalkyl, C₁₋₃alkoxy, C₁₋₃haloalkoxy or -CN;
R⁴ is a hydrogen, F, Cl or Br atom or a C₁₋₃alkyl, C₁₋₃haloalkyl, C₁₋₃alkoxy, C₁₋₃haloalkoxy, -CN, -SO₂R⁵, -SO₂N(R⁶)₂, -CON(R⁶)₂, -N(R⁶)₂, -NHSO₂R⁵ or -NHCOR⁵ group;
R⁵ is a C₁₋₃alkyl group;
R⁶, which may be the same or different, is each a hydrogen atom or a C₁₋₃alkyl group; and
R^{a} and R^{b}, which may be the same or different, is each an atom or group selected from hydrogen or C₁₋₃alkyl, or R^{a} and R^{b} may be joined to form a C₃₋₆cycloalkyl or heterocycloalkyl group as defined for R¹ and R²;
and the salts, solvates, hydrates, tautomers, isomers or *N*-oxides thereof.

### Description of the Invention

It will be appreciated that certain compounds of formula (1) may exist as geometric isomers (E or Z isomers). The compounds may also have one or more chiral centres, and exist as enantiomers or diastereomers. The invention is to be understood to extend to all such geometric isomers, enantiomers, diastereomers and mixtures thereof, including racemates. Formula (1) and the formulae hereinafter are intended to represent all individual isomers and mixtures thereof, unless stated or shown otherwise. In addition, compounds of formula (1) may exist as tautomers, for example keto (CH₂C=O)-enol (CH=CHOH) tautomers.

It will also be appreciated that where desired the compounds of the invention may be administered in a pharmaceutically acceptable pro-drug form, for example as a protected hydroxamic acid derivative, e.g. as either *N*- or *O-*substituted derivatives, such as *O*-benzoyl. It will be further appreciated that the pro-drugs may be converted *in vivo* to the active compounds of formula (1), and the invention is intended to extend to such pro-drugs.

In the compounds of the invention as represented by formula (1) and the more detailed description hereinafter certain of the general terms used in relation to substituents are to be understood to include the following atoms or groups unless specified otherwise.

Thus as used herein the term "C₁₋₆alkyl", whether present as a group or part of a group, refers to straight or branched C₁₋₆alkyl groups such as methyl, ethyl, *n*-propyl, isopropyl, *n*-butyl, isobutyl, *sec*-butyl, *tert*-butyl or neopentyl. The term "C₁₋₃alkyl" refers to a straight or branched C₁₋₃alkyl group selected from methyl, ethyl, *n*-propyl or isopropyl.

The term "C₃₋₆cycloalkyl group" refers to non-aromatic cyclic, saturated C₃₋₆ ring systems selected from cyclopropyl, cyclobutyl, cyclopentyl or cyclohexyl.

The term "heterocycloalkyl group" refers to a 3- to 10-membered saturated monocyclic or multicyclic hydrocarbon ring system containing one, two, or three L² linker atoms or groups. Particular examples of suitable L² atoms or groups include -O-, -S- and -N(R¹¹)-, where R¹¹ is a hydrogen atom or a C₁₋₆ alkyl group.

Particular examples of heterocycloalkyl groups include 3- to 7-membered monocyclic ring systems such as azetidinyl, tetrahydrofuranyl, tetrahydropyranyl, tetrahydrothiophenyl, tetrahydrothiopyranyl, pyrrolidinyl, oxazolidinyl, dioxolanyl, e.g. 1,3-dioxolanyl, imidazolidinyl, pyrazolidinyl, thiazolidinyl, piperidinyl, 1,4-dioxanyl, morpholinyl, 1,4-dithianyl, thiomorpholinyl, piperazinyl, *N*-C₁₋₆alkyl-piperazinyl, *N*-C₁₋₆alkylpyrrolidinyl, *N*-C₁₋₆alkylpiperidinyl, *N*-C₁₋₆alkylmorpholinyl, homopiperazinyl or 7- to 10-membered multicyclic ring systems such as quinuclidinyl or 1,4-dioxaspiro[4.5]decanyl.

Typical heterocycloalkyl groups which may represent either R¹ and R² when joined together or R^{a} and R^{b} when joined together include 3- to 7-membered monocyclic ring systems, such as azetidinyl, tetrahydrofuranyl, tetrahydropyranyl, pyrrolidinyl and piperidinyl.

Heterocycloalkyl groups may be linked to the remainder of the compound of formula (1) by any available carbon atom or, when part of the group -C₁₋₆alkylheterocycloalkyl, by any carbon atom or heteroatom, e.g. nitrogen atom, as appropriate.

The term "halogen atom" is intended to include fluorine, chlorine, bromine or iodine atoms.

The term "C₁₋₆haloalkyl" is intended to include the C₁₋₆alkyl groups as defined herein substituted by one, two or three of the halogen atoms just described. Similarly the term "C₁₋₃haloalkyl" is intended to include the C₁₋₃alkyl groups as defined herein substituted by one, two or three of the halogen atoms just described. Particular examples of such groups include -CF₃, -CCl₃, -CHF₂, -CHCl₂, -CH₂F and -CH₂Cl groups.

The term "C₁₋₆alkoxy" as used herein refers to straight or branched C₁₋₆alkoxy groups such as methoxy, ethoxy, *n*-propoxy, isopropoxy or *tert*-butoxy. Likewise the term "C₁₋₃alkoxy" as used herein refers to straight or branched C₁₋₃alkoxy groups such as methoxy, ethoxy, *n*-propoxy or isopropoxy.

The term "C₁₋₆haloalkoxy" as used herein includes any of those C₁₋₆alkoxy groups as defined herein substituted by one, two or three halogen atoms as described above. Similarly the term "C₁₋₃haloalkoxy" includes any of those C₁₋₃alkoxy groups as defined herein substituted by one, two or three halogen atoms as described above. Particular examples include -OCF₃, -OCCl₃, -OCHF₂, -OCHCl₂, -OCH₂F and -OCH₂Cl groups.

The term "aryl" refers to an aromatic carbocyclic radical having a single ring or two condensed rings. This term includes, for example, phenyl and naphthyl.

The term "heteroaryl" refers to a 5- to 10-membered aromatic monocyclic or multicyclic hydrocarbon ring system in which one, two or three atoms in the ring system is an element other than carbon, chosen from amongst nitrogen, oxygen or sulfur (or oxidised versions thereof, such as N-oxide). Monocyclic heteroaryl groups include, for example, five- or six-membered heteroaryl groups containing one, two or three heteroatoms selected from oxygen, sulfur or nitrogen atoms.

Particular examples of monocyclic ring heteroaryl groups of this type include pyrrolyl, furyl, thienyl, imidazolyl, *N*-C₁₋₆alkylimidazolyl, oxazolyl, isoxazolyl, thiazolyl, isothiazolyl, pyrazolyl, triazolyl, oxadiazolyl, thiadiazolyl, pyridyl, pyrimidinyl, pyridazinyl, pyrazinyl, tetrazolyl, triazinyl and pyridyl-*N*-oxide.

Particular examples of bicyclic ring heteroaryl groups of this type include benzofuryl, benzothienyl, indolyl, benzimidazolyl, benzothiazolyl, benzoxazolyl, benzisoxazolyl, quinazolinyl, quinoxalinyl, naphthyridinyl, pyrido[3,4-*b*]pyridyl, pyrido[3,2-*b*]pyridyl, pyrido[4,3-*b*]pyridyl, quinolinyl and isoquinolinyl.

The heteroaryl groups may be attached to the remainder of the compound of formula (1) by any available carbon atom.

The terms "-C₁₋₆alkylaryl", "-C₁₋₆alkylheteroaryl", "-C₁₋₆alkyl-heterocycloalkyl" and "-C₁₋₆alkylC₃₋₆cycloalkyl" refer to a C₁₋₆alkyl group as defined herein in which a terminal hydrogen atom therein is replaced by an aryl, heteroaryl, heterocycloalkyl or C₃₋₆cycloalkyl group as described herein.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isothionates, arylsulphonates, e.g. *p*-toluenesulphonates, besylates or napsylates, phosphates, sulphates, hydrogensulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

One group of compounds of formula (1) has the formula (2): 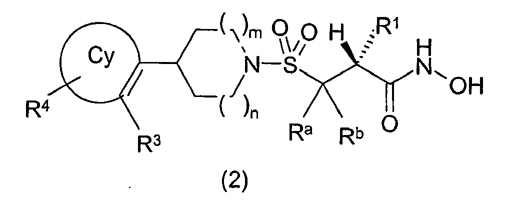 wherein m, n, Cy, R^{a}, R^{b}, R¹, R³ and R⁴ are as defined herein for compounds of formula (1);
and the salts, solvates, hydrates, tautomers, isomers or *N*-oxides thereof.

In one particular group of compounds of the invention Cy is a phenyl group or a monocyclic heteroaryl group, especially pyridyl, pyrimidinyl or pyrazinyl.

Cy is typically a phenyl group.

Another group of compounds of formula (1) has the formula (3): 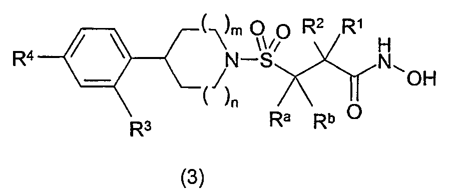 wherein m, n, R^{a}, R^{b}, R¹, R², R³ and R⁴ are as defined herein for compounds of formula (1);
and the salts, solvates, hydrates, tautomers, isomers or *N*-oxides thereof.

One particular group of compounds of formula (3) has the formula (4): 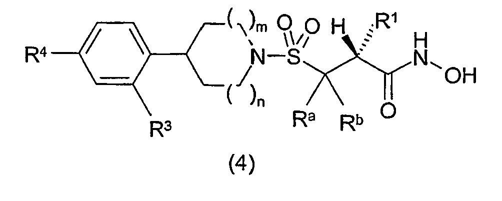 wherein m, n, R^{a}, R^{b}, R¹, R³ and R⁴ are as defined herein;
and the salts, solvates, hydrates, tautomers, isomers or N-oxides thereof.

In another particular aspect of the invention R^{a} and R^{b} is each a hydrogen atom.

In another particular aspect of the invention m is the integer 1 and n is zero or the integer 1.

In one group of compounds of formula (1), (2), (3) or (4) n is preferably the integer 1. In compounds of this type m is especially the integer 1.

R² in one particular group of compounds of the invention is a hydrogen atom.

R¹ in one group of compounds of formula (1), (2), (3) or (4) is a group selected from C₁₋₆alkyl, phenyl, heteroaryl, heterocycloalkyl, C₃₋₆cycloalkyl, -(CH₂)₁₋₂phenyl, -(CH₂)₁₋₂heteroaryl, -(CH₂)₁₋₂heterocycloalkyl or -(CH₂)₁₋₂C₃₋₆cycloalkyl, in which each phenyl or heteroaryl group, present as or as part of the group R¹, may optionally be substituted with 1, 2 or 3 substituents selected from the group R⁷, as herein defined; and in which each alkyl, heterocycloalkyl or cycloalkyl group, present as or as part of the group R¹, may optionally be substituted with 1, 2 or 3 substituents selected from the group R⁸, as herein defined.

R¹ in a further group of compounds of formula (1), (2), (3) or (4) is a group selected from optionally substituted C₁₋₆alkyl, phenyl, heterocycloalkyl, C₃₋₆cycloalkyl or -(CH₂)₁₋₂phenyl.

Particular R¹ examples include C₁₋₆alkyl, e.g. isopropyl, phenyl, pyridyl, pyrimidinyl, pyrrolyl, furyl, thienyl, imidazolyl, *N*-C₁₋₆alkylimidazolyl, oxazolyl, isoxazolyl, thiazolyl, isothiazolyl, tetrahydropyranyl, tetrahydrofuranyl, piperidinyl, pyrrolidinyl, 1,4-dioxaspiro[4.5]decanyl, cyclobutyl, cyclopentyl, cyclohexyl, -CH₂phenyl or -CH₂pyridyl.

R¹ in one particular group of compounds of formula (1), (2), (3) or (4) is an isopropyl, phenyl, 3,4-difluorophenyl, tetrahydropyranyl, cyclopentyl, -CH₂phenyl or -(CH₂)-3,4-difluorophenyl group, especially isopropyl, phenyl or -CH₂phenyl. Further typical examples include piperidin-4-yl, 1-methylpiperidin-4-yl, 1-*tert-*butoxycarbonylpiperidin-4-yl, tetrahydropyran-4-yl, cyclopentyl and 3,4-difluorobenzyl.

In one group of compounds of the invention R⁷ is an atom or group selected from F, Cl, Br, C₁₋₆alkyl, C₁₋₆haloalkyl, C₁₋₆alkoxy, C₁₋₆haloalkoxy or -CN.

R⁷ in compounds of the invention may be, for example, an atom or group selected from F, Cl, methyl, -CF₃, -CF₂H, methoxy, -OCF₃, -OCF₂H or -CN. Further examples of the group R⁷ include -CO₂H, -CO₂CH₃, -CO₂CH₂CH₃, -CO₂C(CH₃)₃, -CONH₂, -CON(H)CH₃, -CON(CH₃)₂ or -COCH₃. In one particular aspect of the invention R⁷ is a F atom.

In one group of compounds of the invention R⁸ is an atom or group selected from F, C₁₋₆alkyl, C₁₋₆haloalkyl, C₁₋₆alkoxy, C₁₋₆haloalkoxy, =O or =NOR¹⁰.

R⁸ in compounds of the invention may be, for example, an atom or group selected from F, methyl, -CF₃, -CF₂H, methoxy, -OCF₃, -OCF₂H, =O, =NOH or =NOCH₃. Further examples of the group R⁸ include -CO₂H, -CO₂CH₃, -CO₂CH₂CH₃, -CO₂C(CH₃)₃, -CONH₂, -CON(H)CH₃, -CON(CH₃)₂ or -COCH₃ groups, especially -CO₂C(CH₃)₃. More particular examples of the group R⁸ include methyl and -CO₂C(CH₃)₃.

Another group of compounds of the invention has the formula (1) or (3) wherein R¹ and R² together with the carbon atom to which they are attached form a C₃₋₆cycloalkyl group, particularly cyclobutyl, optionally substituted with R⁹ as defined herein.

In one group of compounds of the invention R⁹ is an atom or group selected from F, C₁₋₆alkyl, C₁₋₆haloalkyl, C₁₋₆alkoxy, C₁₋₆haloalkoxy, =O or =NOR¹⁰.

R⁹, in one group of compounds of the invention, is an atom or group selected from F, methyl, -CF₃, -CF₂H, methoxy, -OCF₃, -OCF₂H, =O, =NOH or =NOCH₃.

Particular R³ examples include F, Cl, methyl, ethyl, isopropyl, -CF₃, -CF₂H, methoxy, ethoxy, -OCF₃, -OCF₂H or -CN. R³, in one group of compounds of formula (1), (2), (3) or (4), is a F atom or a methyl, -CF₃, methoxy or -OCF₂H group. R³ may typically also be a Cl atom.

Particular R⁴ examples include hydrogen, F, Cl, methyl, ethyl, isopropyl, -CF₃, -CF₂H, methoxy, ethoxy, -OCF₃, -OCF₂H, -CN, -SO₂CH₃, -SO₂N(H)₂, -SO₂N(CH₃)₂, -SO₂N(H)CH₃, -CON(H)₂, -CON(CH₃)₂, -CON(H)CH₃, -N(H)₂, -N(CH₃)₂, -N(H)CH₃, -NHSO₂CH₃ and -NHCOCH₃. R⁴, in one group of compounds of formula (1), (2), (3) or (4), is a hydrogen, F or Cl atom or a methyl, -CF₃, methoxy or -OCF₂H group, especially a hydrogen, fluorine or chlorine atom.

Certain compounds of the invention also have a surprisingly good selectivity for CD23 when compared with their ability to inhibit matrix metalloproteinases. Examples of such matrix metalloproteinases include MMP 9 and MMP 13. Such compounds are particularly useful for the treatment of diseases in which CD23 has a role, for example allergic and other diseases as described herein. Compounds of the invention which have this useful property include those of formulae (1), (2), (3) or (4) wherein R³ is an atom or group selected from F, Cl, C₁₋₃alkyl or C₁₋₃alkoxy. An especially preferred group of compounds is where R³ is a C₁₋₃alkyl, particularly methyl, or C₁₋₃alkoxy, particularly methoxy, group.

Particular compounds of this type include:
2-[4-(2-methoxyphenyl)piperidine-1-sulfonylmethyl]-*N*-hydroxy-3-methylbutyramide;
2-[4-(2-methyl-4-fluorophenyl)piperidine-1-sulfonylmethyl]-*N*-hydroxy-3-methylbutyramide;
2-benzyl-*N*-hydroxy-3-[4-(2-methoxyphenyl)piperidine-1-sulfonyl]propionamide;
2-benzyl-*N*-hydroxy-3-[4-(2-methylphenyl)piperidine-1-sulfonyl]propionamide;
*N*-hydroxy-3-[4-(2-methoxyphenyl)piperidine-1-sulfonyl]-2-phenylpropionamide;
2(R)-[4-(2-methoxyphenyl)piperidine-1-sulfonylmethyl]-*N*-hydroxy-3-methylbutyramide;
2(*R*)-[4-(2-methylphenyl)piperidine-1-sulfonylmethyl]-*N*-hydroxy-3-methylbutyramide;
1-[4-(2-methoxyphenyl)piperidine-1-sulfonylmethyl]cyclobutane carboxylic acid hydroxyamide;
1-[4-(2-methylphenyl)piperidine-1-sulfonylmethyl]cyclobutane carboxylic acid hydroxyamide;
and the salts, solvates, hydrates, tautomers, isomers or *N*-oxides thereof.

Compounds of formula (1), (2), (3) or (4) are potent inhibitors of CD23 shedding. The ability of the compounds to act in this way may be simply determined by employing tests such as those described in the Examples hereinafter. The selectivity profile for certain compounds of the invention with respect to their inhibition of matrix metalloproteinases may be determined using the assay as described in Example D in the International Patent Application WO-A-98/05635.

Thus the compounds of the invention may be used in the treatment of conditions associated with increased levels of sCD23. The invention extends to such a use and in general to the use of the compounds of formula (1), (2), (3) or (4) for the manufacture of a medicament for treating such diseases and disorders.

Particular uses to which the compounds of the invention may be put include allergic diseases such as asthma, atopic dermatitis and other atopic diseases, allergic rhinitis, gastrointestinal allergies such as food allergies, eosinophilia, conjunctivitis, glomerular nephritis, graft-v-host disease, systemic anaphylaxis or hypersensitivity responses, urticaria, shock, drug allergies, insect sting allergies or parasite infections.

In a particular embodiment, the compounds of the present invention are useful for the treatment of the aforementioned exemplary disorders irrespective of their etiology, for example for the treatment of asthma, atopic dermatitis or allergic rhinitis.

Compounds of the invention may also be of use in other diseases where sCD23 is implicated including inflammatory diseases, such as rheumatoid arthritis or psoriasis, and neoplastic diseases, such as lymphoma or leukemia.

The compounds of formula (1), (2), (3) or (4) can be used alone or in combination with other compounds having related utilities to prevent and treat allergic disorders and diseases, including asthma and atopic dermatitis, as well as those pathologies as discussed herein.

For the prophylaxis or treatment of disease the compounds according to the invention may be administered as pharmaceutical compositions, and according to a further aspect of the invention we provide a pharmaceutical composition which comprises a compound of formula (1), (2), (3) or (4) together with one or more pharmaceutically acceptable carriers, excipients or diluents.

Alternative compositions of this invention comprise a compound of formula (1), (2), (3) or (4) or a salt thereof; an additional agent selected from an immunosuppressant or an anti-inflammatory agent; and any pharmaceutically acceptable carrier, adjuvant or vehicle.

Pharmaceutical compositions according to the invention may take a form suitable for oral, buccal, parenteral, nasal, topical, vaginal or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogenphosphate); lubricants (e.g. magnesium stearate, talc or silica); disintegrants (e.g. potato starch or sodium glycolate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, and flavouring, colouring or sweetening agents, as appropriate.

Preparations for oral administration may be suitably formulated to give controlled release of the active compound.

For buccal administration the compositions may take the form of tablets or lozenges formulated in conventional manner.

The compounds of formula (1), (2), (3) or (4) may be formulated for parenteral administration by injection, e.g. by bolus injection or infusion. Formulations for injection may be presented in unit dosage form, e.g. in glass ampoules or multi-dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use. For particle-mediated administration the compounds of formula (1), (2), (3) or (4) may be coated on particles such as microscopic gold particles.

In addition to the formulations described above, the compounds of formula (1), (2), (3) or (4) may also be formulated as a depot preparation. Such long-acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas or mixture of gases.

For vaginal or rectal administration the compounds of formula (1), (2), (3) or (4) may be formulated as a suppository. These formulations may be prepared by mixing the active ingredient with a suitable non-irritating excipient which is a solid at room temperature but liquid at the body temperature. Such materials include, for example, cocoa butter and polyethylene glycols.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular condition will vary depending on the compound chosen, and the condition of the patient to be treated. In general, however, daily dosages may range from around 100 ng/kg to 100 mg/kg, e.g. around 0.01 mg/kg to 40 mg/kg body weight, for oral or buccal administration, from around 10 ng/kg to 50 mg/kg body weight for parenteral administration, and around 0.05 mg to around 1000 mg, e.g. around 0.5 mg to around 1000 mg, for nasal administration or administration by inhalation or insufflation.

The compounds according to the present invention may be used as pharmacological standards for use in the development of new biological tests and in the search for new pharmacological agents. The compounds according to the present invention may also be radiolabelled.

The compounds of the invention may be prepared by a number of processes as generally described below and more specifically in the Examples hereinafter. Many of the reactions described are well-known standard synthetic methods which may be applied to a variety of compounds and as such can be used not only to generate compounds of the invention but also, where necessary, the intermediates thereto.

In the following process description, the symbols m, n, Cy, R^{a}, R^{b}, R¹, R², R³ and R⁴, when used in the formulae depicted, are to be understood to represent those groups described above in relation to formula (1), (2), (3) or (4) unless otherwise indicated. In the reactions described below, it may be necessary to protect reactive functional groups, for example hydroxy, amino, thio or carboxy groups, where these are desired in the final product, to avoid their unwanted participation in the reactions. Conventional protecting groups may be used in accordance with standard practice [see, for example, Greene, T. W. in "Protective Groups in Organic Synthesis", John Wiley and Sons (1999) and the examples herein]. In some instances, deprotection may be the final step in the synthesis of a compound of formula (1), (2), (3) or (4) and the processes according to the invention described hereinafter are to be understood to extend to such removal of protecting groups.

Thus, according to a further aspect of the invention, a compound of formula (1), or particular isomers thereof, may be prepared using the general method shown in Scheme A:

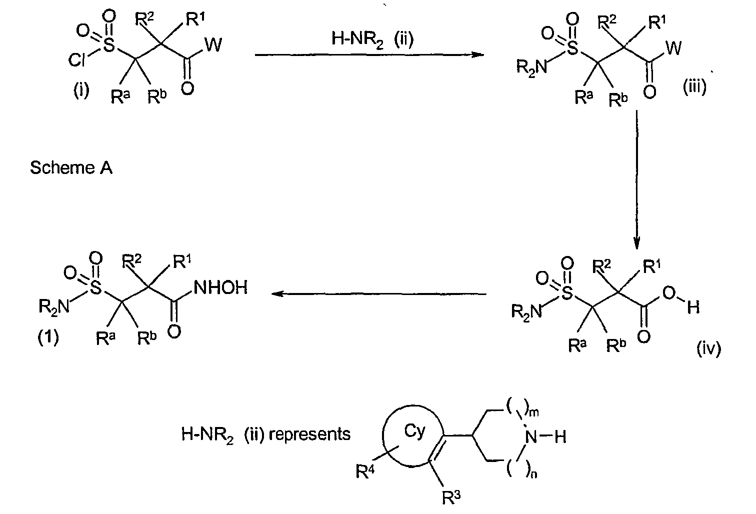

Thus, compounds of formula (iii), where W is, for example, an alkoxy group, such as methoxy, ethoxy or *tert*-butoxy, or a chiral auxiliary, for example 4(*R*)-benzyloxazolidin-2-one, may be prepared by methods well known in the literature, for example by reaction of a sulfonyl chloride (i) with an amine (ii) in the presence of an amine base, such as triethylamine, in a halogenated solvent, such as dichloromethane, at room temperature.

Compounds of general formula (i) are either known or may be made by one skilled in the art using conditions known in the literature, see for example WO-A-99/24399, or as described in the Examples hereinafter. Compounds of general formula (ii) are available commercially or they be made using methods known in the literature or by any method known to those skilled in.the art.

Carboxylic acids of general formula (iv) may be prepared by deprotection of a suitably protected carboxylic acid of formula (iii). For example, where W is an alkoxy group, such as ethoxy, a base such as aqueous lithium hydroxide may be used. Alternatively, trifluoroacetic acid may be used when W is a *tert-*butyl group; or in the case of a chiral auxiliary, such as 4(*R*)-benzyloxazolidin-2-one, lithium hydroxide/hydrogen peroxide may be used. Appropriate solvent and temperature conditions, such as those described in the Examples hereinafter, may be used.

Hydroxamic acids of general formula (1) may be prepared using conditions well known in the literature. For example, treatment of an acid of formula (iv) with oxalyl chloride in an inert solvent (such as dichloromethane) gives an intermediate acid chloride, which may or may not be isolated, but which in turn is reacted with hydroxylamine at a suitable temperature such as room temperature to give the desired hydroxamic acid (1). Alternatively, an acid of formula (iv) may be activated *in situ* using, for example, a diimide such as 1-(3-dimethylaminopropyl)-3-ethylcarbodiimide hydrochloride, advantageously in the presence of a catalyst such as a *N*-hydroxy compound, e.g. *N* hydroxybenzotriazole, using suitable conditions, e.g. in *N*,*N*-dimethylformamide at -15°C, prior to the subsequent addition of a suitably protected hydroxylamine, such as *tert-*butyldimethylsilyl hydroxylamine, and warming to ambient temperature. The protecting group may be removed using appropriate conditions, such as water or tetrabutylammonium fluoride and acetic acid in tetrahydrofuran at 0°C, to yield the desired hydroxamic acid of formula (1).

Intermediates of formulae (i)-(iv) and any other intermediates required to obtain compounds of formula (1), (2), (3) or (4), when not available commercially, may be prepared by methods known to those skilled in the art following procedures set forth in references such as Rodd's *Chemistry of Carbon Compounds,* Volumes 1-15 and Supplementals (Elsevier Science Publishers, 1989), Fieser and Fieser's *Reagents for Organic Synthesis,* Volumes 1-19 (John Wiley and Sons, 1999), *Comprehensive Heterocyclic Chemistry,* Ed. Katritzky *et al.,* Volumes 1-8, 1984, and Volumes 1-11, 1994 (Pergamon), *Comprehensive Organic Functional Group Transformations,* Ed. Katritzky *et al.,* Volumes 1-7, 1995 (Pergamon), *Comprehensive Organic Synthesis,* Ed. Trost and Fleming, Volumes 1-9 (Pergamon, 1991), *Encyclopedia of Reagents for Organic Synthesis,* Ed. Paquette, Volumes 1-8 (John Wiley and Sons, 1995), Larock's *Comprehensive Organic Transformations* (VCH Publishers Inc., 1989), and March's *Advanced Organic Chemistry* (John Wiley and Sons, 1992).

Thus, for example, an amine of general formula (ii), in particular where Cy is a phenyl group, may be prepared using methods known to those skilled in the art, including the general methods as shown in Scheme B:

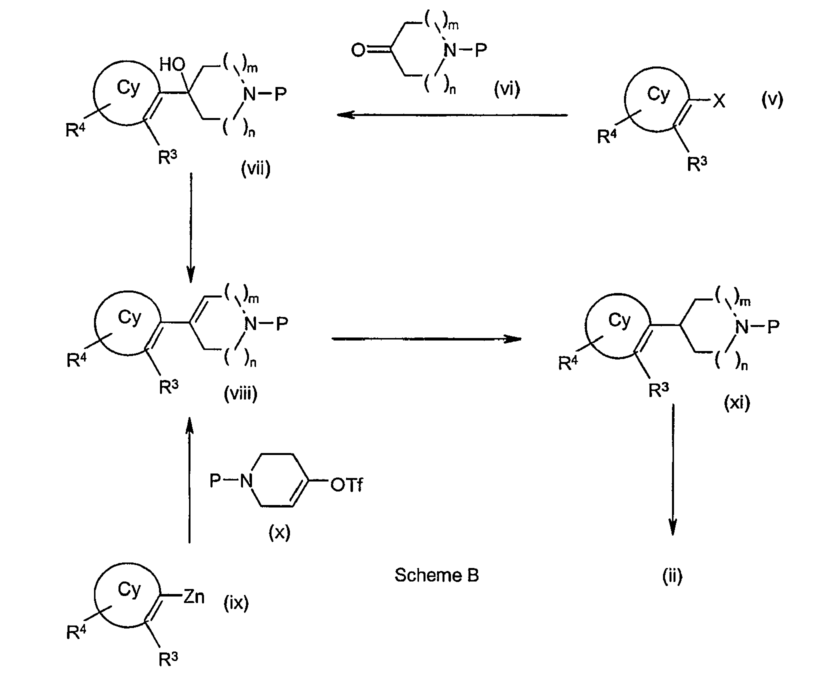

Thus, where appropriate, a leaving group X, e.g. an aromatic halogen substituent (e.g. X = Br), in the compounds of general formula (v) may be subjected to halogen-metal exchange by treatment with a base, for example a lithium base such as *n*-butyl- or *tert*-butyllithium, optionally at a low temperature, e.g. around -78°C, in a solvent such as tetrahydrofuran and then quenched with a ketone of general formula (vi) (where P is a suitable protecting group, such as carbobenzyloxy) to give an alcohol of formula (vii). The alcohol thus formed may then be dehydrated using standard conditions, such as acid catalysis, to yield a compound of formula (viii).

Alternatively, a compound of formula (viii) may be prepared by reaction of a zinc species, e.g. an aryl-zinc species of formula (ix), with a triflate of formula (x) in the presence of a catalyst, such as a palladium-containing catalyst, using conditions known to those skilled in the art.

The compound of formula (viii) may then be reduced using standard methodology, such as palladium-catalysed hydrogenation, to yield a compound of formula (xi), containing a protecting group P, which may be converted to a compound of formula (ii) using standard deprotection methods. It will be appreciated by those skilled in the art that different protecting groups P may be required at each stage of the synthesis in order to satisfy the reaction conditions and as such they may be interconverted using standard methods.

A compound of formula (ii) wherein m and n are both the integer 1 may also be prepared from a compound of formula (xii): 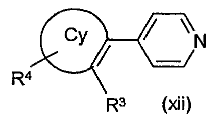 by selective hydrogenation of the pyridine ring, for example using a palladium or nickel catalyst under a hydrogen atmosphere. The compound of general formula (xii) may be prepared using methods known to those skilled in the art, such as standard biaryl coupling methodology.

It will be appreciated that compounds of formula (1), (2), (3) or (4) or any preceding intermediates may be further derivatised by one or more standard synthetic methods employing substitution, oxidation, reduction or cleavage reactions. Particular substitution approaches include conventional alkylation, arylation, heteroarylation, acylation, thioacylation, halogenation, sulphonylation, nitration, formylation and coupling procedures. It will be appreciated that these methods may also be used to obtain or modify other compounds of formula (1), (2), (3) or (4) or any preceding intermediates where appropriate functional groups exist in these compounds.

Salts of compounds of formula (1), (2), (3) or (4) may be prepared by reaction of a compound of formula (1), (2), (3) or (4) with an appropriate base or acid in a suitable solvent or mixture of solvents, e.g. an organic solvent such as an ether, e.g. diethyl ether, or an alcohol, e.g. ethanol, or an aqueous solvent, using conventional procedures. Salts of compounds of formula (1), (2), (3) or (4) may be exchanged for other salts by use of conventional ion-exchange chromatography procedures.

Where it is desired to obtain a particular enantiomer of a compound of formula (1), (2), (3) or (4) this may be produced from a corresponding mixture of enantiomers using any suitable conventional procedure for resolving enantiomers.

Thus, for example, diastereomeric derivatives, e.g. salts, may be produced by reaction of a mixture of enantiomers of formula (1), (2), (3) or (4), e.g. a racemate, and an appropriate chiral compound, e.g. a chiral base. The diastereomers may then be separated by any convenient means, for example by crystallisation, and the desired enantiomer recovered, e.g. by treatment with an acid in the instance where the diastereomer is a salt.

In another resolution process a racemate of formula (1), (2), (3) or (4) may be separated using chiral High Performance Liquid Chromatography. Alternatively, if desired, a particular enantiomer may be obtained by using an appropriate chiral intermediate in one of the processes described above.

Chromatography, recrystallisation and other conventional separation procedures may also be used with intermediates or final products where it is desired to obtain a particular geometric isomer of the invention.

The following Examples illustrate the invention. All temperatures are in °C. Where experimental detail is not given for the preparation of a reagent it is either commercially available, or it is known in the literature, for which the CAS number is quoted. The compounds are named with the aid of Beilstein Autonom supplied by MDL Information Systems GmbH, Theodor-Heuss-Allee 108, D-60486 Frankfurt, Germany.

¹H NMR spectra were obtained at 300 MHz or 400 MHz unless otherwise indicated.

The following LCMS conditions were used to obtained the retention times (RT) as described herein:

### LCMS conditions:

HP1100 (Diode Array) linked to a Finnigan LC-Q Mass Spectrometer, ESI mode with Pos/Neg ionisation.
Column: Luna C18(2) 100 × 4.6 mm, 5 µm particle size Analytical column
Column Temp: 35°C
Mobile Phase: A: Water + 0.08% formic acid
B: Acetonitrile + 0.1% formic acid
Flow rate: 3 ml/min
Gradient:

| Time (min): | % Composition B: |
|---|---|
| 0 | 5 |
| 4.4 | 95 |
| 5.30 | 95 |
| 5.32 | 5 |
| 6.5 | 5 |

Run time: 6.5 min
Typical Injection Volume: 5 µl
Detector Wavelength: DAD 205-330 nm

### Preparative LC conditions:

Gilson 215 liquid handler setup.
Column: Luna C18(2) 250 × 21.2 mm, 5 µm particle size PREP column
Column Temp: Ambient
Mobile Phase: A: Water + 0.08% formic acid
   B: Acetonitrile + 0.1% formic acid
Gradient: Variable - depends on retention of sample in LCMS screen
Run Time: 20 min
Flow rate: 20 ml/min
Typical Injection Volume: 750 µl of 25 mg/ml solution
Detector Wavelength: 210 and 254 nm

Abbreviations used:

| | |
|---|---|
| DCM - dichloromethane | THF - tetrahydrofuran |
| MeOH - methanol | DMF - *N,N*-dimethylformamide |
| TFA - trifluoroacetic acid | MTBE - *tert*-butyl methyl ether |
| nBuLi - *n*-butyllithium | Hunig's base - *N,N*-diisopropylethylamine |
| CDCl₃ - deuterated chloroform | d₆DMSO - deuterated dimethylsulfoxide |
| Methanol-d₄ - deuterated methanol | |

### Intermediate 1

### 3-Methyl-2-methylenebutyric acid

Isopropyl malonic acid (30 g) was dissolved in 1,4-dioxane (200 ml) and piperidine (30 ml) was added, followed by aqueous formaldehyde (30 ml). The solution was stirred overnight and the resulting thick white suspension was heated to 100°C for 2 h, then cooled and evaporated. The mixture was diluted with water (400 ml) and washed with ether (200 ml), then acidified with citric acid to pH 4 and extracted with DCM (200 ml). The solvent was washed with water (200 ml) and brine (200 ml), dried and evaporated to give the title compound as a colourless solid (25 g). MS 114 (M).

### Intermediate 2

### 2-Bromomethyl-3-methylbutyric acid

3-Methyl-2-methylenebutyric acid (25 g) was dissolved in 48% hydrobromic acid in acetic acid (100 ml) and the solution stirred overnight at room temperature, then added to water (300 ml) and extracted with diethyl ether (2 x 200 ml). The solvent washed with water (200 ml) and brine (200 ml), dried and evaporated to give the title compound as a pale amber solid (33 g). MS 195 (M).

### Intermediate 3

### 2-Bromomethyl-3-methylbutyric acid tert-butyl ester

2-Bromomethyl-3-methylbutyric acid (33 g) was placed in a Parr pressure reactor, cooled to -78°C, and isobutylene (200 ml) and DCM (200 ml) were added, followed by concentrated sulphuric acid (1 ml). The vessel was sealed and the mixture stirred at room temperature for 18 h, then pressure carefully released and the solution added to saturated sodium bicarbonate solution (400 ml). The mixture was extracted with diethyl ether (2 x 200 ml), the solvent washed with water (200 ml) and brine (200 ml) and evaporated *in vacuo* to give the title compound as a colourless liquid (33 g). MS 251 (M).

### Intermediate 4

### 2-Acetylsulfanylmethyl-3-methylbutyric acid tert-butyl ester

Potassium thioacetate (20 g) was added to a solution of 2-bromomethyl-3-methylbutyric acid *tert-*butyl ester (33 g) in DMF (200 ml) and the brown mixture stirred for 18 h, then added to water (1 litre), and the mixture extracted with diethyl ether (300 ml). The solvent was washed with water, saturated sodium bicarbonate solution and brine, dried and evaporated to give the title compound as an amber oil (29 g). MS 246 (M).

### Intermediate 5

### 2-Chlorosulfonylmethyl-3-methylbutyric acid tert-butyl ester

Chlorine was passed through a solution of 2-acetylsulfanylmethyl-3-methylbutyric acid *tert*-butyl ester (29 g) in DCM (100 ml) and water (100 ml) at 0°C for 1 h, giving a pale green solution. The phases were separated and the organic layer washed with water (200 ml), sodium bicarbonate solution (200 ml) and brine (200 ml), dried and evaporated to give the product as a colourless liquid which crystallised on refrigeration (27 g). MS 271 (M).

### Intermediate 6

### 2-Benzylacrylic acid

Prepared from benzyl malonic acid (25 g), using the method as described for 3-methyl-2-methylenebutyric acid, to give the title compound as white solid (18 g). MS 162 (M + 1).

### Intermediate 7

### 2-Bromomethyl-3-phenylpropionic acid

Prepared from 2-benzylacrylic acid (18 g), using the method as described for 2-bromomethyl-3-methylbutyric acid, to give the title compound as a white solid (23 g). MS 243 (M).

### Intermediate 8

### 2-Bromomethyl-3-phenylpropionic acid tert-butyl ester

Prepared using the method as described for 2-bromomethyl-3-methylbutyric acid *tert-*butyl ester from 2-bromomethyl-3-phenylpropionic acid (23 g) to give the title compound as a brown oil (28 g). MS 299 (M).

### Intermediate 9

### 2-Acetylsulfanylmethyl-3-phenylpropionic acid tert-butyl ester

Prepared using the method as described for 2-acetylsulfanylmethyl-3-methylbutyric acid *tert*-butyl ester from 2-bromomethyl-3-phenylpropionic acid *tert*-butyl ester (28 g) to give the title compound as a yellow oil (18.5 g). MS 294 (M).

### Intermediate 10

### 2-(Chlorosulfonylmethyl)-3-phenylpropionic acid tert-butyl ester

Prepared using the method as described for 2-chlorosulfonylmethyl-3-methylbutyric acid *tert*-butyl ester from 2-acetylsulfanylmethyl-3-phenylpropionic acid *tert*-butyl ester (18.5 g) as a colourless solid (19 g). MS 319 (M + H).

### Intermediate 11

### 1-(Chlorosulfonylmethyl)cyclobutane carboxylic acid ethyl ester

*n*-Butyllithium (49.8 ml of 1.6M solution in hexanes) was added to a solution of diisopropylamine (11.2 ml) in THF (90 ml) at -78°C and the solution stirred for 30 min. A solution of ethyl cyclobutanecarboxylate (10 ml) was added dropwise and the mixture stirred for 30 min, then treated with diiodomethane (6.4 ml). The mixture was stirred for 3 h and allowed to warm to room temperature, quenched with water (50 ml) and evaporated. The residual mixture was partitioned between water and ethyl acetate, the organic layer washed with water and brine, dried and evaporated. The residue was dissolved in DMF (50 ml) and potassium thioacetate (8.3 g) was added. The brown solution was stirred overnight at room temperature, then added to water and extracted with ethyl acetate. The solvent was washed with water (200 ml) and brine (200 ml), dried and evaporated to a brown oil. The residue was dissolved in DCM (100 ml), water (100 ml) was added and chlorine bubbled through the mixture at 0°C. The organic layer was washed with water (200 ml) and brine (200 ml), dried and evaporated to give the title compound as a brown oil (9.8 g). TLC R_{f} 0.45 (2:1 heptane-ethyl acetate).

### Intermediate 12

### 4(R)-Benzyl-3-(3-methylbutyryl)oxazolidin-2-one

*n*-Butyllithium (2.5M in hexanes, 65 ml) was added to a solution of (R)-benzyloxazolidinone (28.9 g) in THF (200 ml) at -78°C and the mixture was stirred for 30 min, then 3-methylbutanoyl chloride (22 ml) was added and the solution stirred for 2 h. The reaction mixture was quenched with saturated ammonium chloride, evaporated *in vacuo* and the residue extracted with DCM (2 x 200 ml). The solvent was washed with water (200 ml), bicarbonate solution (200 ml) and brine (200 ml), dried and evaporated to give the title compound as a colourless solid (41.5 g). MS 261 (M).

### Intermediate 13

### 4(R)-Benzyl-3-(2(S)-hydroxymethyl-3-methylbutyryl)oxazolidin-2-one

Titanium tetrachloride (18 ml) was added to a solution of 4(R)-benzyl-3-(3-methylbutyryl)oxazolidin-2-one (41.5 g) in DCM at 0°C. Hunig's base (28 ml) was added and the purple solution stirred for 30 min, then a solution of trioxane (11.2 g) in DCM was added dropwise, followed by titanium tetrachloride. The mixture was stirred vigorously for 2 h at 0°C, giving an amber solution, which was quenched with saturated aqueous ammonium chloride. The phases were separated and the organic layer washed with water (150 ml), bicarbonate solution (150 ml), and brine (150 ml), dried and evaporated to a white solid (45 g). MS 291 (M).

### Intermediate 14

### 4(R)-Benzyl-3-(2(R)-iodomethyl-3-methylbutyryl)oxazolidin-2-one

Iodine (42 g), triphenylphosphine (47 g) and imidazole (12 g) were added to a solution of 4(*R*)-benzyl-3-(2(*S*)-hydroxymethyl-3-methylbutyryl) oxazolidin-2-one (45 g) in toluene (500 ml) and the mixture was boiled under reflux for 1 h. The resulting suspension was cooled, filtered and the filtrate washed with water (150 ml), and brine (150 ml). The solid residue was dissolved in DCM and filtered through silica (200g) eluting with ether/hexane to give the title compound as a pale yellow oil (57 g). MS 401 (M).

### Intermediate 15

### 4(R)-Benzyl-3-(2(R)-acetylthiomethyl-3-methylbutyryl)oxazolidin-2-one

Potassium thioacetate (19 g) was added to a solution of 4(R)-benzyl-3-(2(*R*)-iodomethyl-3-methylbutyryl)oxazolidin-2-one (56 g) in DMF (300 ml) and the mixture was stirred at room temperature for 3 h, then added to water (2 l) and extracted with ether (2 x 500 ml). The solvent was washed with water (400 ml), bicarbonate solution (200 ml) and brine (200 ml), dried and evaporated to give the title compound as a pale amber oil (49 g). MS 349 (M).

### Intermediate 16

### 4(R)-Benzyl-3-(2(R)-chlorosulfonylmethyl-3-methylbutyryl)oxazolidin-2-one

Chlorine was bubbled through a solution of 4(*R*)-benzyl-3-(2(*R*)-acetylthiomethyl-3-methylbutyryl)oxazolidin-2-one (49 g) in DCM (200 ml) and water (200 ml) until the solution became yellow. The mixture was stirred vigorously for 1 h, then purged with nitrogen, the phases were separated and the organic layer washed with water (150 ml), and brine (150 ml), dried and evaporated to give the title compound as a colourless gum (42 g). MS 373 (M). 1H NMR (δH, CDCl₃) 7.20-7.40 (5H, m), 4.65-4.80 (2H, m), 4.45 (1H, dd), 4.20 (2H, d), 3.70 (1H, dd), 3.45 (1H, dd), 2.65 (1H, dd), 2.10 (1H, m), 1.15 (3H, d), 0.03 (3H, d).

### Intermediate 17

### 3-Bromo-2-phenylpropionic acid

Prepared from phenylmalonic acid [CAS number 492-38-6] (4 g) following the procedure as described for 2-bromomethyl-3-methylbutyric acid to yield an amber oil (5.2 g). MS 229 (M).

### Intermediate 18

### 3-Bromo-2-phenylpropionic acid tert-butyl ester

Prepared using the method as described for 2-bromomethyl-3-methylbutyric acid *tert*-butyl ester from 3-bromo-2-phenylpropionic acid (5 g) as a colourless oil (4.5 g). MS 285 (M).

### Intermediate 19

### 3-Acetylsulfanyl-2-phenylpropionic acid tert-butyl ester

Prepared using the method as described for 2-acetylsulfanylmethyl-3-methylbutyric acid *tert*-butyl ester from 3-bromo-2-phenylpropionic acid *tert*-butyl ester (4 g) as a yellow liquid (3.3 g). MS 280 (M).

### Intermediate 20

### 3-Chlorosulfonyl-2-phenylpropionic acid tert-butyl ester

Prepared using the method as described for 2-chlorosulfonylmethyl-3-methylbutyric acid *tert*-butyl ester from 3-acetylsulfanyl-2-phenylpropionic acid *tert*-butyl ester (3 g) as a beige solid (2.1 g). TLC R_{f} 0.47 (ether).

### Intermediate 21

### 1-tert-Butoxycarbonylpiperidin-4-ylmalonic acid

Titanium tetrachloride (22 ml) was added dropwise to a solution of 1-*tert-*butoxycarbonylpiperidin-4-one (20 g) and diethyl malonate (16 ml) in THF (200 ml) at 0°C. Pyridine (52 ml) was added dropwise and the mixture was stirred overnight. Water (500 ml) and EtOAc (500 ml) were added, the organic layer washed with brine (300 ml) and 1M HCl (300 ml), dried and evaporated. The residue was dissolved in EtOH (200 ml) and hydrogenated at atmospheric pressure over 10% Pd/C (2 g) overnight. The mixture was filtered and aqueous NaOH (2M, 200 ml) was added. The solution was boiled under reflux for 6 h, cooled, evaporated and the residue partitioned between 1M HCl (400 ml) and EtOAc (400 ml). The solvent was dried (MgSO₄) and evaporated and the residue triturated with ether to give the title compound as white crystalline solid (9 g). TLC R_{f} 0.27 (EtOAc/1% AcOH).

### Intermediate 22

### 2-[1-(tert-Butoxycarbonyl)piperidin-4-yl]acrylic acid

Intermediate 21 (9 g) was dissolved in 1,4-dioxane (60 ml) and formaldehyde solution (37% aq., 10 ml) and piperidine (10 ml) were added. The mixture was stirred overnight, then heated at reflux for 1 h. The solution was evaporated *in vacuo* and partitioned between 1M HCl (100 ml) and Et₂O (100 ml). The solvent was washed with water (50 ml) and brine (50 ml), dried and evaporated to give the title compound as colourless crystalline solid (5.6 g). TLC R_{f} 0.42 (Et₂O).

### Intermediate 23

### 4-[1-(4(R)-Benzyl-2-oxooxazolidine-3-carbonyl)vinyl]piperidine-1-carboxylic acid tert-butyl ester

Intermediate 22 (4.0 g) was dissolved in DCM (50 ml) and pyridine (3 ml) and treated with oxalyl chloride (3 ml) and DMF (1 drop). The solution was stirred for 3 h, then evaporated *in vacuo* and azeotroped to dryness with heptane. The product was dissolved in THF (20 ml) and added dropwise to a solution of (*R*)-benzyloxazolidin-2-one (2.7 g) and nBuLi (2.5M in hexanes, 6.5 ml) in THF (60 ml) at -78°C. The mixture was stirred for 4 h, then quenched with ammonium chloride solution (200 ml), extracted with EtOAc (200 ml) and the solvent washed with water (50 ml) and brine (50 ml), dried (MgSO₄) and evaporated. The residue was columned on silica (3:1 ether-hexane) to give the title compound as a colourless solid (3.3 g). TLC R_{f} 0.35 (3:1 ether-hexanes). 1H NMR (δH, CDCl₃) 7.20-7.40 (5H, m), 5.40 (2H, m), 4.75 (1H, m). 4.10-4.35 (4H, m), 3.30 (1H, dd), 2.85 (1H, dd), 2.70 (2H, dt), 2.55 (1H, dt), 1.85 (2H, dt), 1.60 (9H, s), 1.45-1.60 (2H, m).

### Intermediate 24

### 4-[1(R)-Acetylsulfanylmethyl-2-(4(R)-benzyl-2-oxooxazolidin-3-yl)-2-oxoethyl]piperidine-1-carboxylic acid tert-butyl ester

Intermediate 23 (3.3 g) was stirred in thioacetic acid (10 ml) for 18 h at room temperature. The mixture was diluted with Et₂O (100 ml) and washed with 1M NaOH (2 x 50 ml), water and brine (50 ml), dried (MgSO₄) and evaporated. Analysis showed the crude product to be a 9 to 1 mixture of diastereomers. The residue was columned (1:1 Et₂O/hexane) to give the title compound as a white solid (2.6 g). TLC R_{f} 0.27 (1:1 Et₂O/hexane). 1H NMR (δH, CDCl₃) 7.20-7.40 (5H, m), 4.70 (1H, m), 4.00-4.20 (5H, m), 3.25-3.40 (2H, m), 3.10 (1H, dd), 2.75 (1H, dd), 2.55-2.70 (2H, m), 2.35 (3H, s), 1.90 (1H, m), 1.20-1.70 (4H, m), 1.45 (9H, s).

### Intermediate 25

### 4-[2-(4(R)-Benzyl-2-oxooxazolidin-3-yl)-1-(R)-chlorosulfonylmethyl-2-oxoethyl]piperidine-1-carboxylic acid tert-butyl ester

Chlorine was bubbled through a solution of Intermediate 24 (1.6 g) and sodium acetate (5 g) in DCM (50 ml) and water (20 ml) at 0°C for 10 min, until a faint yellow colour persisted in the organic layer. The mixture was stirred for a further 30 min, then the phases were separated and the organic layer washed with water (50 ml) and brine (50 ml), dried (MgSO₄) and evaporated to give the title compound as a colourless solid (1.6 g). TLC R_{f} 0.53 (Et₂O). 1H NMR (δH, CDCl₃) 7.20-7.40 (5H, m), 4.80 (1H, m), 4.70 (1H, m), 4.45 (1H, dd), 4.15-4.30 (4H, m), 3.80 (1H, dd), 3.50 (1H, dd), 2.65 (1H, dd), 2.55-2.70 (2H, m), 1.90 (1H, m), 1.70 (2H, m), 1.45 (9H, s), 1.35-1.55 (2H, m).

### Intermediate 26

### (Tetrahydropyran-4-ylidene)acetic acid methyl ester

Carbomethoxy triphenylphosphonium bromide (45 g) was added to a solution of tetrahydropyran-4-one (10 g) in THF (100 ml). Sodium hydride (4.2 g) was added carefully in small portions. The suspension was stirred at reflux for 18 h, then cooled, filtered and evaporated. The residue was filtered through silica, eluting with Et₂O/hexane 1:1 to give the title compound as a colourless oil (13 g). MS 156 (M).

### Intermediate 27

### (Tetrahydropyran-4-yl)acetic acid methyl ester

Intermediate 26 (13 g) was hydrogenated at atmospheric pressure in MeOH (100 ml) for 24 h, the solution filtered and evaporated to give the title compound as a colourless liquid (13 g). MS 158 (M).

### Intermediate 28

### (Tetrahydropyran-4-yl)acetic acid

Sodium hydroxide (16 g) in water (400 ml) was added to a solution of Intermediate 27 (13 g) in MeOH (60 ml). The mixture was stirred overnight at room temperature, then evaporated *in vacuo.* The solution was washed with Et₂O (50 ml), acidified with concentrated hydrochloric acid to pH 2 and extracted with EtOAc (100 ml), the solvent washed with brine (50 ml), dried (MgSO₄) and evaporated to give the title compound as a colourless solid (10.2 g). MS 144 (M).

### Intermediate 29

### 4(R)-Benzyl-3-[2-(tetrahydropyran-4-yl)acetyl]oxazolidin-2-one

Oxalyl chloride (5 ml) and DMF (1 drop) were added to a solution of Intermediate 28 (10 g) in DCM (100 ml). The mixture was stirred for 3 h, then evaporated *in vacuo* and thoroughly azeotroped with toluene. The residue was dissolved in THF (30 ml) and added dropwise to a solution of (R)-benzyloxazolidinone (12.1 g) and nBuLi (2.5M in hexanes, 30 ml) in THF (200 ml) at -78°C. The solution was stirred for 2 h, then quenched with saturated aqueous ammonium chloride (100 ml) and evaporated *in vacuo.* The mixture was extracted with EtOAc (100 ml), solvent washed with water (100 ml) and brine, dried (MgSO₄) and evaporated to give the title compound as a colourless solid (14 g). MS 304 (M + H).

### Intermediate 30

### 4(R)-Benzyl-3-[3-hydroxy-2(S)-(tetrahydropyran-4-yl)propionyl]oxazolidin-2-one

Titanium tetrachloride (14 ml, 1M in DCM) was added to a solution of Intermediate 29 (4 g) in DCM (100 ml) at 0°C, followed by Hunig's base (2.5 ml). The mixture was stirred for 30 min, then trioxane (1.2 g) and titanium tetrachloride (14 ml) were added. The dark purple suspension was stirred for 4 h, then quenched with saturated ammonium chloride solution, the organic layer washed with water (50 ml) and brine (50 ml), dried (MgSO₄) and evaporated. The residue was columned on silica (Et₂O) to give the title compound as a white solid (1.6 g). MS 334 (M + 1).

### Intermediate 31

### 4(R)-Benzyl-3-[3-iodo-2(R)-(tetrahydropyran-4-vl)propionyl]oxazolidin-2-one

Intermediate 30 (1.6 g) was dissolved in toluene (30 ml) and triphenyl phosphine (1.4 g), iodine (1.3 g) and imidazole (350 mg) were added. The mixture was stirred at reflux for 1 h, then cooled, washed with water (50 ml) and the solution evaporated. The residue was columned on silica (2:1 Et₂O:hexane) to give the title compound as a white solid (1.8 g). MS 444 (M + 1).

### Intermediate 32

### 4(R)-Benzyl-3-[3-acetylsulfanyl-2(R)-(tetrahydropyran-4-yl)propionyl]-oxazolidin-2-one

Intermediate 31 (1.8 g) was dissolved in DMF (10 ml) and potassium thioacetate (600 mg) was added. The suspension was stirred for 4 h, then added to water (100 ml) and extracted with EtOAc (50 ml). The solvent was washed with water (2 x 30 ml), bicarbonate (50 ml) and brine (50 ml), dried (MgSO₄) and evaporated to give the title compound as a pale orange gum (1.5 g). MS 392 (M + H).

### Intermediate 33

### 3-(4(R)-Benzyl-2-oxooxazolidin-3-yl)-3-oxo-2(R)-(tetrahydropyran-4-yl)propane-1-sulfonyl chloride

Chlorine was passed through a solution of Intermediate 32 (1.5 g) in DCM (100 ml) and water (100 ml) for 30 min. The solution was stirred for 30 min, purged with nitrogen and the phases separated. The organic layer was washed with water (50 ml) and brine (50 ml), dried (MgSO₄) and evaporated to give the title compound as a colourless solid (1.3 g). MS 416 (M + 1).

### Intermediate 34

### 4-(2-Chloro-4-fluorophenyl)piperidine trifluoroacetate

2-Chloro-4-fluorophenyl zinc iodide (2.9 g) in THF (30 ml) was added dropwise to a solution of 4-(trifluoromethanesulphonyloxy)-1-*tert*-butoxycarbonyl-1,2,3,6-tetrahydropyridine (2 g) and tetrakis(triphenylphosphine)palladium(0) (0.33 g) in THF (30 ml). The solution was stirred at 50°C for 3 h, then the mixture was poured into sodium bicarbonate solution (100 ml) and extracted with DCM (100 ml). The solvent was dried (MgSO₄) and evaporated. The product was dissolved in MeOH (100 ml) and hydrogenated over platinum oxide catalyst (0.10 g) at atmospheric pressure. The product was dissolved in DCM (20 ml) and TFA (5 ml) was added. The solution was stirred for 2 h, then evaporated and azeotroped with heptane (2 x 50 ml). The crude product was purified by crystallization from MeOH/Et₂O to give the title compound as a colourless solid (0.91 g). MS 214 (M+1).

### Intermediate 35

### 4-(2,4-Dichlorophenyl)piperidine trifluoroacetate

Prepared in a similar manner to the method of Intermediate 34 from 2,4-dichlorophenyl zinc iodide (0.5 M in THF, 20 ml) and 4-(trifluoromethane-sulphonyloxy)-1-*tert-*butoxycarbonyl-1,2,3,6-tetrahydropyidine (3.31 g) as a white solid (1.2 g). MS 231 (M + 1).

### Intermediate 36

### 1-Benzyl-4-(2-methoxy-4-fluorophenyl)tetrahydropyridine

2-Methoxy-4-fluoro-l-bromobenzene (1.61 g) was treated with nBuLi (2.5M in hexanes, 3.2 ml) in Et₂O (100 ml) at -78°C. The solution was stirred for 10 min, then a solution of *N*-benzylpiperidin-4-one (1.52 g) in Et₂O (50 ml) was added dropwise. The mixture was stirred for 2 h, then washed with ammonium chloride solution, dried and evaporated. The crude product was dissolved in toluene (100 ml) and P₂O₅ (3.5 g) was added. The mixture was heated at reflux for 8 h, then washed with 1M NaOH (100 ml), dried (MgSO₄), evaporated and the crude product purified by chromatography on silica (5% MeOH/DCM) to give the title compound as a pale yellow oil (2.21 g). MS 298 (M + 1).

### Intermediate 37

### 1-Benzyl-4-(2-methoxy-4-fluorophenyl)piperidine

Intermediate 36 (2.21 g) was hydrogenated over platinum oxide (20 mg) in MeOH (30 ml) for 18 h. The mixture was filtered and evaporated *in vacuo* to give the title compound as a colourless oil (2.2 g). MS 300 (M + 1).

### Intermediate 38

### 4-(2-Methoxy-4-fluorophenyl)piperidine hydrochloride

Intermediate 37 (1.9 g) was dissolved in dichloroethane (10 ml) and 1-chloroethyl chloroformate (1 g) was added. The solution was heated at reflux for 1 h, then the mixture evaporated. MeOH (20 ml) was added and the solution heated at reflux for 2 h, then cooled and diluted with Et₂O (50 ml). The product was collected by filtration to give the title compound as a colourless solid (1.2 g). MS 210 (M + 1).

### Intermediate 39

### 4-(2-Methoxy-4-chlorophenyl)piperidine hydrochloride

Prepared in a similar manner using the methods as described in Intermediates 36-38 from 2-methoxy-4-chloro-1-bromobenzene (0.86 g) and *N-*benzylpiperidin-4-one (0.74 g) as a colourless solid (300 mg). MS 226 (M + 1).

### Intermediate 40

### 4-(2-Methyl-4-fluorophenyl)piperidine hydrochloride

Prepared in a similar manner using the methods as described in Intermediates 36-38 from 2-methyl-4-fluoro-l-bromobenzene (3.25 g) and *N-*benzylpiperidin-4-one (3.25 g) as a colourless solid (1.2 g). MS 193 (M + 1).

### Intermediate 41

### 2-Cyclopentylacrylic acid

Prepared using the method as described for Intermediate 1, from cyclopentylmalonic acid (5 g), to give the title compound as a yellow oil (4.1 g). 1H NMR (δH, CDCl₃) 11.50 (1H, s), 6.30 (1H, s), 5.80 (1H, s), 2.95 (1H, q), 1.95-2.00 (2H, m), 1.65-1.80 (4H, m), 1.35-1.50 (2H, m).

### Intermediate 42

### 3-Bromo-2-cyclopentylpropionic acid

Prepared using the method as described for Intermediate 2, from Intermediate 41 (4.1 g), to give the title compound as a white solid (4.34 g). 1H NMR (δH, CDCl₃) 10.50 (1H, s), 3.45-3.65 (2H, m), 2.55-2.75 (1H, m), 1.90-2.15 (1H, m), 1.70-1.90 (2H, m), 1.45-1.70 (4H, m), 1.15-1.45 (2H, m). MS 221 (M).

### Intermediate 43

### 3-Acetylsulfanyl-2-cyclopentylpropionic acid

Potassium thioacetate (2.24 g) was added to a solution of Intermediate 42 (4.34 g) in DMF (20 ml) and the mixture stirred for 24 h. The brown solution was added to water (100 ml), extracted with Et₂O (100 ml) and the solvent washed with water and brine, dried (MgSO₄) and evaporated *in vacuo* to give the title compound as a brown solid (3.8 g). 1H NMR (δH, CDCl₃) 3.30 (1H, dd), 2.96-3.00 (1H, m), 2.50 (1H, dd), 2.38 (3H, s), 2.05 (1H, q), 1.85-1.95 (1H, m),1.45-1.70 (4H, m), 1,25-1.40 (2H, m).

### Intermediate 44

### 3-Acetylsulfanyl-2-cyclopentylpropionic acid tert-butyl ester

Intermediate 43 (3.8 g) was dissolved in a mixture of isobutylene (30 ml) and DCM (30 ml), concentrated sulphuric acid (1 ml) was added and the mixture stirred in a Parr pressure reaction vessel for 18 h. The pressure was released cautiously and the solution added to saturated sodium bicarbonate solution, the phases separated and the organic layer washed with water and brine, dried (MgSO₄) and evaporated to give the title compound as a brown oil (4.1 g). 1H NMR (δH, CDCl₃) 3.35 (1H, dd), 3.10-3.25 (1H, m), 2.45 (1H, dd), 2.40 (3H, s), 2.05 (1H, q), 1.85-1.95 (1H, m), 1.40-1.65 (4H, m), 1.30 (9H, s), 1.25-1.40 (2H; m).

### Intermediate 45

### 3-Chlorosulfonyl-2-cyclopentylpropionic acid tert-butyl ester

Prepared using the method as described for Intermediate 5 from Intermediate 44 (1.7 g) to give the title compound as an amber oil (1.6 g). 1H NMR (δH, CDCl₃) 4.25 (1H, dd), 3.70 (1H, dd), 2.90 (1H, dt), 2.05 (1H, m), 1.85-1.95 (1H, m), 1.40-1.65 (4H, m), 1.30 (9H, s), 1.25-1.40 (2H, m).

### Intermediate 46

### 4-(R)-Benzyl-3-[3-(3,4-difluorophenyl)propionyl]oxazolidin-2-one

3,4-Difluorophenylhydrocinnamic acid (10 g, 53 mmol) was dissolved in DCM (100 ml) and stirred with oxalyl chloride (10 ml) and DMF (1 drop) for 3 h at room temperature. The solution was evaporated *in vacuo* and azeotroped with heptane (2 x 200 ml). The residue was dissolved in THF (20 ml) and added dropwise to a solution of (R)-benzyloxazolidinone (9 g) and nBuLi (1.6M in hexanes, 35 ml) in THF (100 ml) at -78°C. The mixture was stirred for 2 h, quenched with saturated ammonium chloride solution (100 ml), evaporated *in vacuo* and the solid product collected by filtration to give the title compound as a colourless solid (16 g). MS 346 (M + 1). TLC R_{f} 0.65 (Et₂O).

### Intermediate 47

### 4(R)-Benzyl-3-[2(R)-hydroxymethyl-3-(3,4-difluorophenyl)propionyl]-oxazolidin-2-one

Intermediate 46 (6.9 g) was dissolved in dry DCM (150 ml) at 0°C and titanium tetrachloride (2.2 ml) was added, followed by Hunig's base (3.5 ml). The mixture was stirred for 30 min, then a solution of trioxane (2.5 g) in DCM (10 ml) was added, followed by titanium tetrachloride (2.2 ml). The solution was stirred for 2 h, then quenched with saturated ammonium chloride (100 ml). The phases were separated and the organic layer washed with bicarbonate solution (2 x 100 ml) and brine, dried and evaporated and the residue columned (3:1 Et₂O /hexanes) to give the title compound as a colourless solid (4.3 g). MS 376 (M + 1). TLC R_{f} 0.45 (Et₂O).

### Intermediate 48

### 4(R)-Benzyl-3-[2(R)-iodomethyl-3-(3,4-difluorophenyl)propionyl]oxazolidin-2-one

Intermediate 47 (4.3 g) was suspended in toluene (100 ml) and triphenylphosphine (3 g), iodine (2.9 g) and imidazole (1 g) were added. The mixture was heated at reflux for 1 h, cooled and washed with water (100 ml), bicarbonate solution (100 ml) and brine, dried and evaporated. The residue was filtered through a silica plug eluting with Et₂O-hexane (1:1) to give the title compound as a colourless gum (3.7 g). TLC R_{f} 0.35 (1:1 Et₂O-hexane).

### Intermediate 49

### 4(R)-Benzyl-3-[2(R)-acetylsulphanylmethyl-3-(3,4-difluorophenyl)propionyl]-oxazolidin-2-one

Intermediate 48 (3.7 g) was dissolved in DMF (50 ml) and potassium thioacetate (0.95 g) was added. The mixture was stirred at room temperature for 3 h, added to water and extracted with Et₂O. The solution was washed with water (2 x 50 ml), and the residue columned (2:1 Et₂O-hexane), to give the title compound as a pale yellow oil (3.05 g). TLC R_{f} 0.45 (2:1 Et₂O-hexane).

### Intermediate 50

### 4(R)-Benzyl-3-[2(R)-chlorosulphonylmethyl-3-(3,4-difluorophenyl)-propionyl]oxazolidin-2-one

Intermediate 49 (3.05 g) was dissolved in DCM (50 ml) and water (40 ml) and chlorine was bubbled through the solution at 0°C for 10 min. The pale yellow mixture was stirred for 30 min, then the phases separated and the organic layer washed with water (50 ml) and brine (50 ml), dried (MgSO₄) and evaporated to give the title compound as a colourless solid (3.10 g). TLC Rf 0.54 (Et₂O). 1H NMR (δH, CDCl₃) 6.90-7.30 (8H, m), 5.00 (1H, m), 4.60 (1H, m), 4.40 (1H, dd), 4.10-4.20 (2H, m), 3.60 (1H, dd), 3.40 (1H, dd), 3.20 (1H, dd), 2.70-2.80 (2H, m).

### Method A

### Example 1

### 2-[4-(2-Ethoxyphenyl)piperidine-1-sulfonylmethyl]-N-hydroxy-3-methylbutyramide

4-(2-Ethoxyphenyl)piperidine [CAS 100617-80-9] (100 mg) was added to a solution of 2-chlorosulfonylmethyl-3-methylbutyric acid *tert*-butyl ester (120 mg) in DCM (10 ml) and triethylamine (50 mg). The solution was stirred for 18 h, then washed with citric acid solution, water and brine, the solvent dried and evaporated. The residue was redissolved in DCM (10 ml) and TFA (2 ml) added. The solution was stirred for 3 h, then evaporated and azeotroped to dryness, the residue dissolved in DCM (10 ml) and washed with water (20 ml) and brine (20 ml). Oxalyl chloride (200 mg) and DMF (1 drop) were added, the solution stirred for 3 h, then evaporated to dryness. The residue was dissolved in THF (10 ml) and aqueous hydroxylamine (0.5 ml) added. The mixture was stirred for 2 h, diluted with water (10 ml) and evaporated to remove THF. The aqueous mixture was extracted with DCM (20 ml), the solvent washed with water (10 ml) and brine (7 ml), dried and evaporated and the residue recrystallised from ether-hexane to give the title compound as a white solid. MS 399 (M + H). 1H NMR (δH, CDCl₃) 8.90 (2H, br s), 7.20 (2H, m), 6.80-7.00 (2H, m), 4.10 (2H, q), 3.80 (2H, m), 3.50 (1H, dd), 2.80-3.10 (4H, m), 2.50 (1H, m), 1.70-2.10 (5H, m), 1.30 (3H, t), 1.00 (6H, appears as triplet).

### Example 2

### 2-[4-(2-Chlorophenyl)piperidine-1-sulfonylmethyl]-N-hydroxy-3-methylbutyramide

Prepared using Method A from 4-(2-chlorophenyl)piperidine [CAS 82211-92-5] (230 mg) and 2-chlorosulfonylmethyl-3-methylbutyric acid *tert*-butyl ester (270 mg) as a white solid (70 mg). MS 389 (M + H). 1H NMR (δH, CDCl₃) 8.50 (2H, br s), 7.10-7.40 (4H, m), 3.90 (2H, m), 3.50 (1H, dd), 3.20 (1H, m), 3.00 (1H, dd), 2.90 (2H, m), 2.40 (1H, m), 1.60-2.00 (5H, m), 1.00 (6H, appears as triplet).

### Example 3

### 2-[4-(2-Methoxy-4-chlorophenyl)piperidine-1-sulfonylmethyl]-N-hydroxy-3-methylbutyramide

Prepared using Method A from 4-(2-methoxy-4-chlorophenyl)piperidine (70 mg) and 2-chlorosulfonylmethyl-3-methylbutyric acid *tert*-butyl ester (80 mg) as a white solid (30 mg). MS 419 (M + H). 1H NMR (δH, CDCl₃) 8.70 (2H, br s), 7.10 (1H, d), 6.85 (1H, d), 6.80 (1H, s), 3.85 (3H, s), 3.70-3.90 (2H, m), 3.50 (1H, dd), 3.00 (2H, m), 2.80-2.90 (2H, m), 2.40 (1H, m), 1.50-2.00 (5H, m), 1.00 (6H, appears as triplet).

### Example 4

### 2-[4-(2-Methyl-4-fluorophenyl)piperidine-1-sulfonylmethyl]-N-hydroxy-3-methylbutyramide

Prepared using Method A from 4-(2-methyl-4-fluorophenyl)piperidine [CAS 277295-96-2] (140 mg) and 2-chlorosulfonylmethyl-3-methylbutyric acid *tert*-butyl ester (160 mg) as a white solid (5.9 mg). MS 387 (M + H). 1H NMR (δH, d₆DMSO) 10.70 (1H, s), 9.00 (1H, s), 7.40 (1H, m), 7.10 (2H, m), 3.70-3.90 (2H, m), 3.60 (1H, dd), 3.10 (1H, dd), 2.80-3.10 (3H, m), 2.40 (3H, s) 2.30-2.40 (1H, m), 1.70-2.00 (5H, m), 1.00 (6H, appears as doublet).

### Example 5

### 2-[4-(2-Difluoromethoxyphenyl)piperidine-1-sulfonylmethyl]-N-hydroxy-3-methylbutyramide

Prepared using Method A from 4-(2-difluoromethoxyphenyl)piperidine (200 mg) and 2-chlorosulfonylmethyl-3-methylbutyric acid *tert*-butyl ester (220 mg) as a white solid (120 mg). MS 421 (M + H). 1H NMR (δH, CDCl₃) 8.60 (2H, br s), 7.20-7.40 (3H, m), 7.10 (1H, d), 6.50 (1H, t), 3.90 (2H, m), 3.50 (1H, dd), 3.10 (1H, tt), 3.00 (1H, dd), 2.80-2.90 (2H, m), 2.40 (1H, dt), 2.00 (1H, m), 1.60-1.90 (4H, m), 1.00 (6H, appears as triplet).

### Example 6

### 2-[4-(2-Fluorophenyl)piperidine-1-sulfonylmethyl]-N-hydroxy-3-methylbutyramide

Prepared using Method A from 2-chlorosulfonylmethyl-3-methylbutyric acid *tert*-butyl ester (270 mg) and 4-(2-fluorophenyl)piperidine (220 mg) as a white solid (130 mg). MS 273 (M + H). 1H NMR (δH, d₆DMSO) 10.60 (1H, s), 8.90 (1H, s), 7.00-7.40 (4H, m) 3.70-3.90 (2H, m), 3.60 (1H, dd), 3.20 (1H, dd), 2.80-3.10 (3H, m), 2.30 (1H, m), 1.80-2.10 (5H, m), 0.95 (6H, appears as doublet).

### Example 7

### 2-[4-(2-Trifluoromethylphenyl)piperidine-1-sulfonylmethyl]-N-hydroxy-3-methylbutyramide

Prepared using Method A from 4-(2-trifluoromethylphenyl)piperidine (270 mg) and 2-chlorosulfonylmethyl-3-methylbutyric acid *tert*-butyl ester (270 mg) as a white solid (160 mg). MS 423 (M + H). 1H NMR (δH, d₆DMSO) 10.80 (1H, s), 9.10 (1H, s), 7.20-7.60 (4H, m) 3.80-4.00 (2H, m), 3.70 (1H, dd), 3.10 (1H, dd), 3.10 (1H, m), 2.80-3.00 (2H, m), 2.40 (1H, m), 1.70-2.00 (5H, m), 1.00 (6H, appears as doublet).

### Example 8

### 2-Benzyl-N-hydroxy-3-[4-(2-trifluoromethylphenyl)piperidine-1-sulfonyl]propionamide

Prepared using Method A from 2-(chlorosulfonylmethyl)-3-phenylpropionic acid *tert*-butyl ester (160 mg) and 4-(2-trifluoromethylphenyl)piperidine (120 mg) as a white solid (8.4 mg) after purification by preparative HPLC. MS 471 (M + 1). 1H NMR (δH, CDCl₃) 8.60 (2H, s), 7.60 (1H, d), 7.40 (1H, t), 7.35 (1H, d), 7.10-7.40 (6H, m), 3.80 (2H, m), 3.60 (1H, dd), 2.60-3.10 (7H, m), 1.60-1.90 (4H, m).

### Example 9

### 2-Benzyl-N-hydroxy-3-[4-(2-fluorophenyl)piperidine-1-sulfonyl]-propionamide

Prepared using Method A from 2-(chlorosulfonylmethyl)-3-phenylpropionic acid *tert*-butyl ester (150 mg) and 4-(2-fluorophenyl)piperidine (100 mg) as a white solid (14 mg) after preparative HPLC. MS 421 (M + 1). 1H NMR (δH, CDCl₃) 8.60 (2H, s), 6.90-7.40 (9H, m), 3.80 (2H, m), 3.60 (1H, dd), 2.60-3.10 (7H, m), 1.60-1.90 (4H, m).

### Example 10

### 2-Benzyl-N-hydroxy-3-[4-(2-methoxyphenyl)piperidine-1-sulfonyl]-propionamide

Prepared using Method A from 2-(chlorosulfonylmethyl)-3-phenylpropionic acid *tert*-butyl ester (150 mg) and 4-(2-methoxyphenyl)piperidine (100 mg) as a white solid (1.2 mg) after preparative HPLC. MS 433 (M + 1). 1H NMR (δH, CDCl₃) 8.50 (2H, br s), 7.20-7.50 (5H, m), 6.80-7.00 (4H, m), 3.80 (3H, s), 3.80 (2H, m), 3.60 (1H, dd), 2.60-3.10 (7H, m), 1.60-1.90 (4H, m).

### Example 11

### 2-Benzyl-N-hydroxy-3-[4-(2-methylphenyl)piperidine-1-sulfonyl]-propionamide

Prepared using Method A from 2-(chlorosulfonylmethyl)-3-phenylpropionic acid *tert*-butyl ester (320 mg) and 4-(2-methylphenyl)piperidine (200 mg) as a white solid (150 mg). MS 417 (M + 1). 1H NMR (δH, CDCl₃) 8.50 (2H, br s), 7.20-7.50 (5H, m), 6.80-7.00 (4H, m), 3.80 (2H, m), 3.60 (1H, dd), 2.60-3.10 (7H, m), 2.40 (3H, s), 1.60-1.90 (4H, m).

### Example 12

### N-Hydroxy-3-[4-(2-Methoxyphenyl)piperidine-1-sulfonyl]-2-phenyl-propionamide

Prepared using Method A from 3-chlorosulfonyl-2-phenylpropionic acid *tert*-butyl ester (230 mg) and 4-(2-methoxyphenyl)piperidine (160 mg) as a beige solid (35 mg). MS 419 (M + 1). 1H NMR (δH, d₆DMSO) 10.90 (1H, s), 8.90 (1H, s), 7.25-7.50 (5H, m), 7.20 (2H, m), 6.90 (1H, d), 6.85 (1H, t), 3.90 (1H, dd), 3.80 (1H, dd), 3.75 (3H, s), 3.60 (2H, m), 3.25 (1H, dd), 2.96 (1H, m), 2.70-2.90 (2H, m), 1.50-1.90 (4H, m).

### Method B

### Example 13

### 2(R)-[4-(2-Methoxyphenyl)piperidine-1-sulfonylmethyl]-N-hydroxy-3-methylbutyramide

4-(2-Methoxyphenyl)piperidine (230 mg) was added to a solution of 4(*R*)-benzyl-3-(2(*R*)-chlorosulfonylmethyl-3-methylbutyryl)oxazolidin-2-one (373 mg) in DCM (10 ml) and triethylamine (200 mg) and the solution was stirred for 2 h at room temperature. The mixture was washed with aqueous citric acid, bicarbonate solution and brine, dried and evaporated. The residue was chromatographed on silica (30% ethyl acetate-hexane) and the product dissolved in THF. Hydrogen peroxide (0.15 ml) was added, the mixture cooled in ice and a solution of lithium hydroxide (40 mg) in water (5 ml) was added dropwise. The mixture was stirred for 2 h, quenched with aqueous sodium sulphite (10% wt/v, 20 ml), then evaporated to half volume *in vacuo.* The aqueous layer was washed with DCM (20 ml), then acidified and extracted with DCM (50 ml). The organic layer was washed with water (20 ml) and brine (20 ml), dried and evaporated. The residue was dissolved in dry DCM (10 ml) and oxalyl chloride (130 mg) was added, followed by one drop of DMF. The solution was stirred for 2 h, evaporated *in vacuo* and azeotroped to dryness. The residue was dissolved in THF (10 ml) and aqueous hydroxylamine (0.5 ml) added, the solution stirred for 2 h, diluted with water (20 ml) and evaporated to remove THF. The solid product was collected by filtration and washed with hexane-MTBE (10 ml) to give the title compound as a white solid (151 mg). MS 385 (M + H). 1H NMR (δH, CDCl₃) 8.50 (2H, br s), 7.15-7.30 (2H, m), 7.00 (1H, t), 6.90 (1H, d), 3.80-3.90 (2H, m), 3.85 (3H, s), 3.60 (1H, dd), 3.00 (1H, m), 2.95 (1H, dd), 2.80-2.90 (2H, m), 2.50 (1H, m), 1.70-2.00 (5H, m), 1.00 (6H, appears as triplet).

### Example 14

### 2(R)-[4-(2-Methylphenyl)piperidine-1-sulfonylmethyl]-N-hydroxy-3-methylbutyramide

Prepared using Method B from 4-(2-methylphenyl)piperidine (170 mg) and 4(*R*)-benzyl-3-(2(*R*)-chlorosulfonylmethyl-3-methylbutyryl)oxazolidin-2-one (370 mg) as a white solid (16 mg). MS 369 (M + H). 1H NMR (δH, d₆DMSO) 10.70 (1H, s), 9.00 (1H, s), 7.10-7.40 (4H, m), 3.70-3.90 (2H, m), 3.50 (1H, dd), 3.10 (1H, dd), 2.80-3.00 (3H, m), 2.50 (1H, m), 2.30 (3H, s), 1.60-1.90 (5H, m), 0.95 (6H, appears as doublet).

### Example 15

### 2(R)-[4-(2-Fluorophenyl)piperidine-1-sulfonylmethyl]-N-hydroxy-3-methylbutyramide

Prepared using Method B from 4(*R*)-benzyl-3-(2(*R*)-chlorosulfonylmethyl-3-methylbutyryl)oxazolidin-2-one (180 mg) and 4-(2-fluorophenyl)piperidine (100 mg) as a beige solid (45 mg). MS 373 (M + H). 1H NMR (δH, d₆DMSO) 10.70 (1H, s), 8.90 (1H, s), 7.10-7.50 (4H, m), 3.60-3.80 (2H, m), 3.50 (1H, dd), 3.10 (1H, dd), 2.80-3.00 (3H, m), 2.40 (1H, dt), 1.60-1.90 (5H, m), 0.96 (6H, appears as doublet).

### Method C

### Example 16

### 1-[4-(2-Methoxyphenyl)piperidine-1-sulfonylmethyl]cyclobutane carboxylic acid hydroxyamide

4-(2-Methoxyphenyl)piperidine (230 mg) was added to a solution of 1-(chlorosulfonylmethyl)cyclobutane carboxylic acid ethyl ester (240 mg) and triethylamine (200 mg) in DCM (20 ml) and the solution stirred at room temperature for 3 h, then washed with water (20 ml) and brine (20 ml), dried and evaporated. The residue was dissolved in methanol (20 ml) and a solution of lithium hydroxide (100 mg) in water (20 ml) was added. The solution was stirred overnight, then evaporated to half volume, acidified with 1M HCl and the mixture extracted with DCM (20 ml). The solvent was washed with water (20 ml) and brine (20 ml), dried and evaporated. The residue was dissolved in DCM (20 ml) and oxalyl chloride (200 mg) added, followed by one drop of DMF. The mixture was stirred for three hours, evaporated and azeotroped to dryness. The residue was dissolved in THF (20 ml) and aqueous hydroxylamine (0.5 ml) was added. The solution was stirred for 3 h, then evaporated *in vacuo.* The residue was triturated with water (10 ml) and the solid product collected by filtration to give the title compound as a white solid (64 mg). MS 383 (M + H). 1H NMR (δH, CDCl₃) 8.50 (2H, m), 7.10-7.30 (2H, m), 6.80-7.00 (2H, m), 3.90 (2H, m), 3.80 (3H, s), 3.50 (2H, s), 3.10 (1H, m), 2.80 (2H, m), 2.35 (2H, m), 2.25 (2H, m), 2.00 (2H, m), 1.70-1.90 (4H, m).

### Example 17

### 1-[4-(2-Methylphenyl)piperidine-1-sulfonylmethyl]cyclobutane carboxylic acid hydroxyamide

Prepared using Method C from 1-(chlorosulfonylmethyl)cyclobutane carboxylic acid ethyl ester (100 mg) and 4-(2-methylphenyl)piperidine (100 mg) to give the title compound as a white solid (7.3 mg). MS 367 (M + 1). 1H NMR (δH, d₆DMSO) 10.50 (1H, s), 8.70 (1H, s), 7.00-7.20 (4H, m), 3.50 (2H, m), 3.40 (2H, s), 2.70 (3H, m), 2.10-2.50 (6H, m), 2.20 (3H, s), 1.50-1.80 (4H, m).

### Example 18

### 2(R)-[4-(2,4-Dichlorophenyl)piperidine-1-sulphonylmethyll-N-hydroxy-3-methylbutyramide

Prepared using the methodology as described in Method A from Intermediate 35 (110 mg) and Intermediate 5 (100 mg) as a white solid (35 mg). MS 424 (M + 1).

### Example 19

### 4-{2-[4-(2,4-Dichlorophenyl)piperidine-1-sulfonyl]-1(R)-hydroxycarbamoylethyl}piperidine-1-carboxylic acid tert-butyl ester

Prepared using the methodology as described in Method B from Intermediate 35 (160 mg) and Intermediate 25 (130 mg) to give the title compound as a white solid (13 mg). MS 565 (M + 1). 1H NMR (d₆DMSO) 10.40 (1H, s), 8.70 (1H, s), 7.20-7.30 (2H, m), 7.00 (1H, m), 3.70 (2H, m), 3.50 (2H, m), 3.25 (1H, dd), 2.90 (1H, dd), 2.80 (1H, m), 2.60 (2H, m), 2.35 (2H, m), 2.20 (1H, m), 0.80-1.60 (9H, m), 1.20 (9H, s).

### Example 20

### 4-{2-[4-(2-Chloro-4-fluorophenyl)piperidine-1-sulfonyl]-1(R)-hydroxy-carbamoylethyl}piperidine-1-carboxylic acid tert-butyl ester

Prepared using the methodology as described in Method B from Intermediate 34 (170 mg) and Intermediate 25 (350 mg) as a white solid (60 mg). MS 546 (M - 1). 1H NMR (d₆DMSO) 10.70 (1H, s), 9.00 (1H, s), 7.20-7.50 (3H, m), 4.00 (2H, m), 3.70 (2H, m), 3.50 (1H, dd). 3.10 (1H, dd), 3.05 (1H, m), 2.95 (2H, m), 2.65 (2H, m), 2.45 (1H, m), 1.00-1.60 (9H, m), 1.40 (9H, s).

### Example 21

### 3-[4-(2-Chloro-4-fluorophenyl)piperidine-1-sulfonyl]-N-hydroxy-2(R)-(piperidin-4-yl)propionamide

In a similar manner to the method described in Example 23 the title compound was prepared from Example 20 (50 mg), TFA (1 ml) and DCM (5 ml). The crude product was purified by preparative HPLC to give the title compound as a colourless solid (6 mg). MS 448 (M + 1). 1H NMR (d₆DMSO) 10.60 (1H, s), 8.90 (1H, s), 6.95-7.20 (3H, m), 3.30 (2H, m), 3.10 (1H, dd), 3.00 (2H, m), 2.80 (1H, dd), 2.75 (1H, m), 2.65 (4H, m), 2.40 (1H, m), 0.85-1.60 (9H, m).

### Example 22

### 3-[4-(2-Chloro-4-fluorophenyl)piperidine-1-sulfonyl]-N-hydroxy-2(R)-(1-methylpiperidin-4-yl)propionamide

Example 21 (60 mg) was dissolved in DCM (10 ml) and treated with formaldehyde (0.2 ml, 37% aq) and sodium triacetoxyborohydride (200 mg) in dichloroethane (20 ml). The mixture was stirred for 2 h, then washed with bicarbonate solution (20 ml), dried (MgSO₄) and evaporated, and the residue recrystallised from EtOAc-hexane, to give the title compound as a colourless solid (32 mg). MS 462 (M + 1). 1H NMR (d₄MeOH) 7.35 (1H, m), 7.20 (1H, m), 7.00 (1H, m), 3.80 (1H, m), 3.65 (1H, dd), 3.30 (2H, m), 3.15 (1H, m), 3.10 (1H, dd), 2.90 (4H, m), 2.40 (1H, m), 2.25 (3H, s), 1.20-2.10 (9H, m).

### Example 23

### 3-[4-(2,4-Dichlorophenyl)piperidine-1-sulfonyl]-N-hydroxy-2(R)-(piperidin-4-yl)propionamide

Example 19 (8 mg) was dissolved in DCM (10 ml) and TFA (1 ml) was added. The solution was stirred for 2 h, then evaporated *in vacuo* and azeotroped to dryness with heptane. The crude product was crystallized from EtOAc-hexanes to give the title compound as a white solid (5 mg). MS 464 (M + 1). 1H NMR (d₆DMSO) 10.60 (1H, s), 8.90 (1H, s), 7.30 (1H, s), 7.20 (1H, d), 7.15 (1H, d), 3.30 (2H, m), 3.10 (1H, dd), 3.00 (2H, m), 2.90 (1H, dd), 2.85 (1H, m), 2.65 (4H, m), 2.40 (1H, m), 0.85-1.60 (9H, m).

### Example 24

### 3-[4-(2-Chloro-4-fluorophenyl)piperidine-1-sulfonyl]-N-hydroxy-2(R)-(tetrahydropyran-4-yl)propionamide

Prepared using the methodology as described for Method B from Intermediate 34 (100 mg) and Intermediate 33 (130 mg) as a white solid (93 mg). MS 449 (M + 1). 1H NMR (d₆DMSO) 10.60 (1H, s), 8.90 (1H, s), 7.20-7.50 (3H, m), 4.00 (2H, m), 3.80 (2H, m), 3.70 (2H, m), 3.60 (2H, m), 3.50 (1H, dd). 3.20 (1H, dd), 3.00 (1H, m), 2.70 (2H, m), 2.40 (1H, m), 1.10-1.60 (9H, m).

### Example 25

### 3-[4-(2-Chloro-4-fluorophenyl)piperidine-1-sulfonyl]-2(R)-(3,4-difluorobenzyl)-N-hydroxypropionamide

Prepared using the methodology as described for Method B from Intermediate 50 (100 mg) and Intermediate 34 (80 mg) as a beige solid (22.7 mg). M + H 491. 1H NMR 6.90-7.30 (6H, m), 3.80 (2H, m), 3.50 (1H, m), 3.10 (1H, m), 3.00 (1H, m), 2.80 (5H, m), 1.60-1.80 (4H, m).

### Example 26

### 2-Cyclopentyl-N-hydroxy-3-(4-o-tolylpiperidine-1-sulfonyl)propionamide

Prepared using the methodology as described for Method A from Intermediate 45 (100 mg) and 4-(2-methylphenyl)piperidine (80 mg) as a beige solid (80 mg). MS 395 (M + 1). 1H NMR 7.00-7.30 (4H, m), 3.70 (2H, m), 3.50 (1H, dd), 3.00 (1H, dd), 2.90 (3H, m), 2.40 (1H, m), 1.20-2.00 (14H, m).

The ability of the compounds of the invention to inhibit the shedding of CD23 may be determined using the following assays:

Abbreviations used:

| | |
|---|---|
| DTT - Dithiothreitol | CO₂ - Carbon Dioxide |
| FCS - Foetal Calf Serum | IL-4 - Interleukin-4 |
| ELISA - Enzyme-Linked ImmunoSorbent Assay | |

### Plasma Membrane CD23 Shedding Assay

Plasma membranes were isolated from RPM18866 cells by initially resuspending the cells in 20 mM Hepes buffer (+ NaCl 150 nM, MgCl₂ 1.5 mM at pH 7.5 containing DTT 1 mM) and homogenising in a glass Dounce homogeniser followed by centrifugation (500 g for 5 min at 4°C) and removal of the supernatant. The homgenisation step was subsequently repeated twice on the remaining cell pellet in order to maximise the yield of membranes. Supernantants were then pooled, further centrifuged (48,000 g for 60 min at 4°C) and finally resuspended in 1 mM sodium bicarbonate. Plasma membranes were further enriched using an aqueous extraction method (Morre, D.J. & Morre, D.M., 1989; *BioTechniques* 7; 9; 946-958).

Plasma membranes were incubated at 37°C in the presence and absence of inhibitor for 2 hours (Marolewski *et al,* 1998; *Biochem. J.;* **333**; 573-579) following which time the reaction was stopped by the addition of 100 µM marimastat. Soluble CD23 shed from the plasma membranes was filtered through a 0.22 µm Millipore filter plate and quantitated by ELISA. IC₅₀ values were calculated by plotting inhibitor concentration versus % inhibition.

The functional effect of the compounds of the invention may be demonstrated using the following assays:

### Cellular CD23 Shedding Assay

The RPMI8866 cell line was routinely grown in RPMI1640 medium containing 10% FCS but was washed twice and resuspended in serum-free RPMl1640 medium immediately prior to the assay. Cells were then plated out in the presence and absence of inhibitor and incubated at 37°C in an atmosphere of 95% air/5% CO₂ for 1 hour (Christie *et al.,* 1997; *Eur. J. Immunol.;* **27;** 3228-3235). Following the time allocated, plates were centrifuged, the supernatants removed and subsequently analysed for shed soluble CD23 by ELISA. IC₅₀ values were calculated by plotting inhibitor concentration versus % inhibition.

### In Vitro Human IgE Synthesis

Mononuclear cells were isolated from human tonsillar tissue over a ficol gradient, washed in PBS and resuspended in RPMI1640 medium containing 10% FCS. Cells were then plated out, stimulated with 20 ng/ml IL-4/5 µg/ml anti-CD40 and incubated in the presence and absence of inhibitor at 37°C in an atmosphere of 95% air/5% CO₂ for 14 days (Christie *et al.,* 1997; *Eur. J. Immunol.;* **27;** 3228-3235). Following the time allocated, plates were centrifuged, the supernatants removed and subsequently analysed for human IgE by ELISA. IC₅₀ values were calculated by plotting inhibitor concentration versus % inhibition.

### Results

The compounds of the accompanying Examples, when tested in the plasma membrane CD23 shedding assay, were all found to possess IC₅₀ values better (i.e. lower) than 1.0 µM.

## Claims

1. A compound of formula (1): 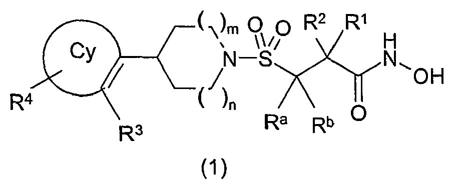 wherein:
Cy is an aryl or heteroaryl group;
m is zero or the integer 1, 2 or 3;
n is zero or the integer 1, 2 or 3; in which the sum of m and n is zero or the integer 1, 2 or 3;
R¹ is a group selected from C₁₋₆alkyl, aryl, heteroaryl, heterocycloalkyl, C₃₋₆cycloalkyl, -C₁₋₆alkylaryl, -C₁₋₆alkylheteroaryl, -C₁₋₆alkylheterocycloalkyl or -C₁₋₆alkylC₃₋₆cycloalkyl, in which each aryl or heteroaryl group, present as or as part of the group R¹, may optionally be substituted with 1, 2 or 3 substituents selected from the group R⁷, wherein each R⁷ may be the same or different, and is an atom or group selected from F, Cl, Br, C₁₋₆alkyl, C₁₋₆haloalkyl, C₁₋₆alkoxy, C₁₋₆haloalkoxy, -CN, -CO₂R^{7a}, -CON(R^{7a})₂ or -COR^{7a}; and in which each alkyl, heterocycloalkyl or cycloalkyl group, present as or as part of the group R¹, may optionally be substituted with 1, 2 or 3 substituents selected from the group R⁸, wherein each R⁸ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R^{7a}, which may be the same or different, is each a hydrogen atom, or a C₁₋₆alkyl or C₁₋₆haloalkyl group;
R^{8a}, which may be the same or different, is each a hydrogen atom, or a C₁₋₆alkyl or C₁₋₆haloalkyl group;
R¹⁰ is a hydrogen atom or a C₁₋₃alkyl group;
R² is a hydrogen atom or a C₁₋₃alkyl group;
or R¹ and R² together with the carbon atom to which they are attached form a C₃₋₆cycloalkyl or heterocycloalkyl group optionally substituted with 1, 2 or 3 substituents selected from the group R⁹, wherein each R⁹ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R³ is an atom or group selected from F, Cl, Br, C₁₋₃alkyl, C₁₋₃haloalkyl, C₁₋₃alkoxy, C₁₋₃haloalkoxy or -CN;
R⁴ is a hydrogen, F, Cl or Br atom or a C₁₋₃alkyl, C₁₋₃haloalkyl, C₁₋₃alkoxy, C₁₋₃haloalkoxy, -CN, -SO₂R⁵, -SO₂N(R⁶)₂, -CON(R⁶)₂, -N(R⁶)₂, -NHSO₂R⁵ or -NHCOR⁵ group;
R⁵ is a C₁₋₃alkyl group;
R⁶, which may be the same or different, is each a hydrogen atom or a C₁₋₃alkyl group; and
R^{a} and R^{b}, which may be the same or different, is each an atom or group selected from hydrogen or C₁₋₃alkyl, or R^{a} and R^{b} may be joined to form a C₃₋₆cycloalkyl or heterocycloalkyl group as defined for R¹ and R²;
and the salts, solvates, hydrates, tautomers, isomers or *N*-oxides thereof.

2. A compound according to Claim 1 which has the formula (2): 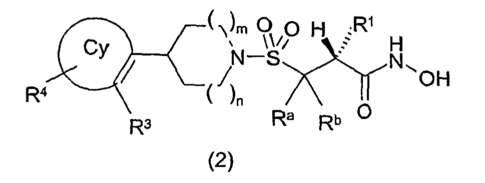 wherein m, n, Cy, R^{a}, R^{b}, R¹, R³ and R⁴ are as defined in Claim 1;
and the salts, solvates, hydrates, tautomers, isomers or *N*-oxides thereof.

3. A compound according to Claim 1 which has the formula (3): 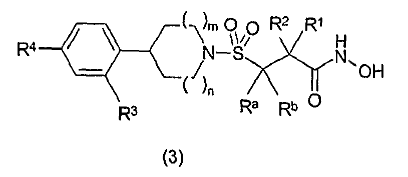 wherein m, n, R^{a}, R^{b}, R¹, R², R³ and R⁴ are as defined in Claim 1;
and the salts, solvates, hydrates, tautomers, isomers or *N*-oxides thereof.

4. A compound according to Claim 3 which has the formula (4): 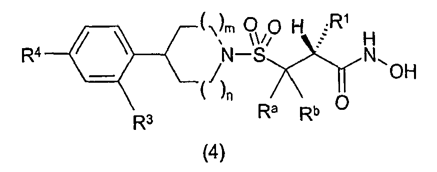 wherein m, n, R^{a}, R^{b}, R¹, R³ and R⁴ are as defined in Claim 1;
and the salts, solvates, hydrates, tautomers, isomers or *N*-oxides thereof.

5. A compound according to Claim 1 or Claim 2 wherein Cy is a phenyl group.

6. A compound according to any preceding Claim wherein R^{a} and R^{b} is each a hydrogen atom.

7. A compound according to any preceding Claim wherein m is the integer 1 and n is zero or the integer 1.

8. A compound of any preceding Claim in which n is the integer 1.

9. A compound of any preceding Claim in which R¹ is a group selected from C₁₋₆alkyl, phenyl, heteroaryl, heterocycloalkyl, C₃₋₆cycloalkyl, -(CH₂)₁₋₂phenyl, -(CH₂)₁₋₂heteroaryl, -(CH₂)₁₋₂heterocycloalkyl or -(CH₂)₁₋₂C₃₋₆cycloalkyl, in which each phenyl or heteroaryl group, present as or as part of the group R¹, may optionally be substituted with 1, 2 or 3 substituents selected from the group R⁷, as defined in Claim 1; and in which each alkyl, heterocycloalkyl or cycloalkyl group, present as or as part of the group R¹, may optionally be substituted with 1, 2 or 3 substituents selected from the group R⁸, as defined in Claim 1.

10. A compound according to any preceding Claim in which R¹ is a group selected from optionally substituted C₁₋₆alkyl, phenyl, heterocycloalkyl, C₃₋₆cycloalkyl or -(CH₂)₁₋₂phenyl.

11. A compound according to any one of Claims 1, 3 or 5 to 8 in which R¹ and R² together with the carbon atom to which they are attached form a C₃₋₆cycloalkyl group optionally substituted with 1, 2 or 3 substituents selected from the group R⁹, as defined in Claim 1.

12. A compound according to Claim 11 in which R¹ and R² together with the carbon atom to which they are attached form a cyclobutyl group.

13. A compound according to any preceding Claim in which R³ is an atom or group selected from F, Cl, methyl, ethyl, isopropyl, -CF₃, -CF₂H, methoxy, ethoxy, -OCF₃, -OCF₂H or -CN.

14. A compound according to any preceding Claim in which R⁴ is an atom or group selected from a hydrogen, F or Cl atom or a methyl, -CF₃, methoxy or -OCF₂H group.

15. A compound of any preceding Claim wherein R³ is an atom or group selected from F, Cl, C₁₋₃alkyl or C₁₋₃alkoxy.

16. A compound according to Claim 15 wherein R³ is a C₁₋₃ alkyl or C₁₋₃alkoxy group.

17. A compound according to Claim 15 or Claim 16 wherein R³ is a methyl or methoxy group.

18. A compound according to claim 1 which is:
2-[4-(2-methoxyphenyl)piperidine-1-sulfonylmethyl]-*N*-hydroxy-3-methylbutyramide;
2-[4-(2-methyl-4-fluorophenyl)piperidine-1-sulfonylmethyl]-*N*-hydroxy-3-methylbutyramide;
2-benzyl-*N*-hydroxy-3-[4-(2-methoxyphenyl)piperidine-1-sulfonyl]propionamide;
2-benzyl-*N*-hydroxy-3-[4-(2-methylphenyl)piperidine-1-sulfonyl]propionamide;
*N*-hydroxy-3-[4-(2-methoxyphenyl)piperidine-1-sulfonyl]-2-phenylpropionamide;
2(*R*)-[4-(2-methoxyphenyl)piperidine-1-sulfonylmethyl]-*N*-hydroxy-3-methylbutyramide;
2(*R*)-[4-(2-methylphenyl)piperidine-1-sulfonylmethyl]-*N*-hydroxy-3-methylbutyramide;
1-[4-(2-methoxyphenyl)piperidine-1-sulfonylmethyl]cyclobutane carboxylic acid hydroxyamide;
1-[4-(2-methylphenyl)piperidine-1-sulfonylmethyl]cyclobutane carboxylic acid hydroxyamide;
and the salts, solvates, hydrates, tautomers, isomers or *N*-oxides thereof.

19. A pharmaceutical composition comprising a compound according to Claim 1 together with one or more pharmaceutically acceptable carriers, excipients or diluents.

## Patentansprüche

1. Verbindung der Formel (1) 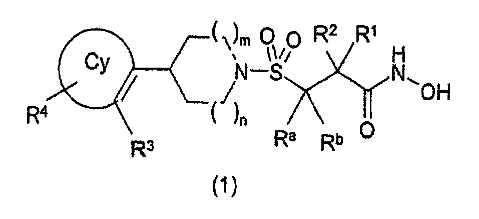 in der
Cy eine Aryl- oder Heteroarylgruppe bedeutet;
m null oder die ganze Zahl 1, 2 oder 3 bedeutet;
n null oder die ganze Zahl 1, 2 oder 3 bedeutet,
wobei die Summe von m und n null oder die ganze Zahl 1, 2 oder 3 ist;
R¹ eine Gruppe aus der Reihe C₁₋₆Alkyl, Aryl, Heteroaryl, Heterocycloalkyl, C₃₋₆Cycloalkyl, -C₁₋₆₋Alkylaryl, -C₁₋₆Alkylheteroaryl, -C₁₋₆₋Alkylheterocycloalkyl oder -C₁₋₆Alkyl-C₃₋₆Cycloalkyl ist, wobei jede Aryl- oder Heteroarylgruppe, die als Gruppe R¹ oder Teil davon vorliegt, gegebenenfalls durch 1, 2 oder 3 Substituenten aus der Gruppe R⁷ substituiert sein kann, wobei jeder Substituent R⁷ gleich oder verschieden sein kann und ein Atom oder eine Gruppe aus der Reihe F, Cl, Br, C₁₋₆Alkyl, C₁₋₆Halogenalkyl, C₁₋₆Alkoxy, C₁₋₆Halogenalkoxy, -CN, -CO₂R^{7a}, -CON(R^{7a})₂ oder -COR^{7a} bedeutet, und wobei jede Alkyl-, Heterocycloalkyl- oder Cycloalkylgruppe, die als Gruppe R¹ oder Teil davon vorliegt, gegebenenfalls durch 1, 2 oder 3 Substituenten aus der Gruppe R⁸ substituiert sein kann, wobei jedes R⁸ gleich oder verschieden sein kann und ein Atom oder eine Gruppe aus der Reihe F, C₁₋₆A1kyl, C₁₋₆Halogenalkyl, C₁₋₆A1koxy, C₁₋₆Halogenalkoxy, =O, =NOR¹⁰, -CO₂R^{8a}, -CON(R^{8a})₂ oder -COR^{8a} bedeutet;
R^{7a}, das gleich oder verschieden sein kann, jeweils ein Wasserstoffatom oder eine C₁₋₆Alkyl- oder C₁₋₆Halogenalkylgruppe bedeutet;
R^{8a}, das gleich oder verschieden sein kann, jeweils ein Wasserstoffatom oder eine C₁₋₆Alkyl- oder C₁₋₆Halogenalkylgruppe bedeutet;
R¹⁰ ein Wasserstoffatom oder eine C₁₋₃Alkylgruppe bedeutet;
R² ein Wasserstoffatom oder eine C₁₋₃Alkylgruppe bedeutet;
oder R¹ und R² gemeinsam mit dem Kohlenstoffatom, an das sie gebunden sind, eine C₃₋₆Cycloalkyl- oder Heterocycloalkylgruppe bilden, die gegebenenfalls durch 1, 2 oder 3 Substituenten aus der Gruppe R⁹ substituiert ist, wobei jedes R⁹ gleich oder verschieden sein kann und ein Atom oder eine Gruppe aus der Reihe F, C₁₋₆Alkyl, C₁₋₆Halogenalkyl, C₁₋₆Alkoxy, C₁₋₆Halogenalkoxy, =O, =NOR¹⁰, -CO₂R^{8a}, -CON (R^{8a})₂ oder -COR^{8a} stammt;
R³ ein Atom oder eine Gruppe aus der Reihe F, Cl, Br, C₁₋₃Alkyl, C₁₋₃Halogenalkyl, C₁₋₃Alkoxy, C₁₋₃Halogenalkoxy oder -CN bedeutet;
R⁴ ein Wasserstoff-, F-, Cl- oder Br-Atom oder eine C₁₋₃Alkyl-, C₁₋₃Halogenalkyl-, C₁₋₃Alkoxy-, C₁₋₃Halogenalkoxy-, -CN-, -SO₂R⁵-, -SO₂N(R⁶)₂-, -CON(R⁶)₂-, -N(R⁶)₂-, -NHSO₂R⁵- oder -NHCOR⁵-Gruppe bedeutet;
R⁵ eine C₁₋₃Alkylgruppe bedeutet;
R⁶, das gleich oder verschieden sein kann, jeweils ein Wasserstoffatom oder eine C₁₋₃Alkylgruppe bedeutet; und
R^{a} und R^{b}, die gleich oder verschieden sein können, jeweils ein Atom oder eine Gruppe aus der Reihe Wasserstoff oder C₁₋₃Alkyl bedeuten, oder R^{a} und R^{b} so verbunden sind, daß sie eine wie für R¹ und R² definierte C₃₋₆Cycloalkyl- oder Heterocycloalkylgruppe bilden;
und ihre Salze, Solvate, Hydrate, Tautomere, Isomere oder *N*-Oxide.

2. Verbindung nach Anspruch 1 mit der Formel (2) 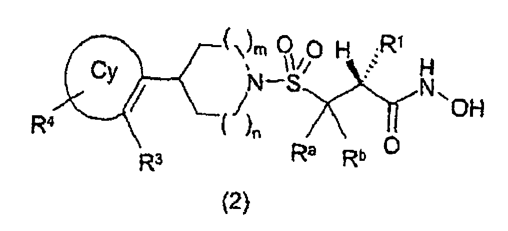 wobei m, n, Cy, R^{a}, R^{b}, R¹, R³ und R⁴ wie in Anspruch 1 definiert sind,
sowie ihre Salze, Solvate, Hydrate, Tautomere, Isomere oder *N*-Oxide.

3. Verbindung nach Anspruch 1 mit der Formel (3) 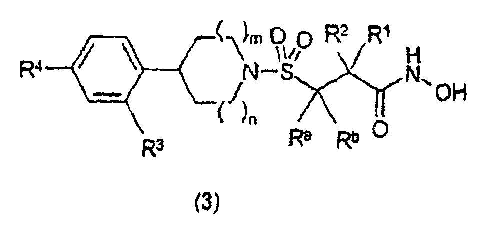 wobei m, n, R^{a}, R^{b}, R¹ ,R², R³ und R⁴ wie in Anspruch 1 definiert sind,
sowie ihre Salze, Solvate, Hydrate, Tautomere, Isomere oder *N*-Oxide.

4. Verbindung nach Anspruch 3 mit der Formel (4) 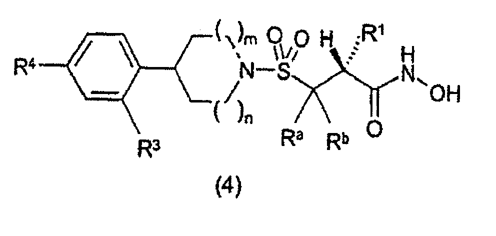 wobei m, n, R^{a}, R^{b}, R¹, R³ und R⁴ wie in Anspruch 1 definiert sind,
sowie ihre Salze, Solvate, Hydrate, Tautomere, Isomere oder *N*-Oxide.

5. Verbindung nach Anspruch 1 oder Anspruch 2, wobei Cy eine Phenylgruppe bedeutet.

6. Verbindung nach einem der vorhergehenden Ansprüche, wobei R^{a} und R^{b} jeweils ein Wasserstoffatom bedeuten.

7. Verbindung nach einem der vorhergehenden Ansprüche, wobei m die ganze Zahl 1 und n null oder die ganze Zahl 1 bedeuten.

8. Verbindung nach einem der vorhergehenden Ansprüche, wobei n die ganze Zahl 1 bedeutet.

9. Verbindung nach einem der vorhergehenden Ansprüche, in der R¹ eine Gruppe aus der Reihe C₁₋₆Alkyl, Phenyl, Heteroaryl, Heterocycloalkyl, C₃₋₆Cycloalkyl, -(CH₂)₁₋₂Phenyl, -(CH₂)₁₋₂Heteroaryl, -(CH₂)₁₋₂Heterocycloalkyl oder -(CH₂)₁₋₂C₃₋₆Cycloalkyl bedeutet, in der jede Phenyl- oder Heteroarylgruppe, die als Gruppe R¹ oder Teil davon vorliegt, gegebenenfalls durch 1, 2 oder 3 Substituenten aus der gemäß 1 definierten Gruppe R⁷ substituiert, und wobei jede Alkyl-, Heterocycloalkyl- oder Cycloalkylgruppe, die als Gruppe R¹ oder als Teil davon vorliegt, gegebenenfalls durch 1, 2 oder 3 Substituenten aus der gemäß Anspruch 1 definierten Gruppe R⁸ substituiert sein kann.

10. Verbindung nach einem der vorhergehenden Ansprüche, wobei R¹ eine Gruppe aus der Reihe gegebenenfalls substituiertes C₁₋₆Alkyl, Phenyl, Heterocycloalkyl, C₃₋₆Cycloalkyl oder -(CH₂)₁₋₂Phenyl bedeutet.

11. Verbindung nach einem der Ansprüche 1, 3 oder 5 bis 8, wobei R¹ und R² gemeinsam mit dem Kohlenstoffatom, an das sie gebunden sind, eine C₃₋₆Cycloalkylgruppe bilden, die gegebenenfalls durch 1, 2 oder 3 Substituenten aus der gemäß Anspruch 1 definierten Gruppe R⁹ substituiert sind.

12. Verbindung nach Anspruch 11, wobei R¹ und R² gemeinsam mit dem Kohlenstoffatom, an das sie gebunden sind, eine Cyclobutylgruppe bilden.

13. Verbindung nach einem der vorhergehenden Ansprüche, wobei R³ ein Atom oder eine Gruppe aus der Reihe F, Cl, Methyl, Ethyl, Isopropyl, -CF₃, -CF₂H, Methoxy, Ethoxy, -OCF₃, -OCF₂H oder -CN bedeutet.

14. Verbindung nach einem der vorhergehenden Ansprüche, wobei R⁴ ein Atom oder eine Gruppe aus der Reihe Wasserstoff-, F- oder Cl-Atom oder Methyl-, -CF₃-, Methoxy- oder -OCF₂H-Gruppe bedeutet.

15. Verbindung nach einem der vorhergehenden Ansprüche, wobei R³ ein Atom oder eine Gruppe aus der Reihe F, Cl, C₁₋₃Alkyl oder C₁₋₃Alkoxy bedeutet.

16. Verbindung nach Anspruch 15, wobei R³ eine C₁₋₃Alkyl- oder C₁₋₃Alkoxygruppe bedeutet.

17. Verbindung nach Anspruch 15 oder Anspruch 16, wobei R³ eine Methyl- oder Methoxygruppe bedeutet.

18. Verbindugng nach Anspruch 1, bei der es sich um
2-[4-(2-Methoxyphenyl)piperidin-1-sulfonylmethyl]-*N*-hydroxy-3-methylbutyramid;
2-[4-(2-Methyl-4-fluorphenyl)piperidin-1-sulfonylmethyl]-*N*-hydroxy-3-methylbutyramid;
2-Benzyl-N-hydroxy-3-[4-(2-methoxyphenyl)piperidin-1-sulfonyl]propionamid;
2-Benzyl-*N*-hydroxy-3-[4-(2-methylphenyl)piperidin-1-sulfonyl]propionamid;
*N*-Hydroxy-3-[4-(2-methoxyphenyl)piperidin-1-sulfonyl]-2-phenylpropionamid;
2(*R*)-[4-(2-Methoxyphenyl)piperidin-1-sulfonylmethyl]-*N*-hydroxy-3-methylbutyramid;
2(*R*)-[4-(2-Methylphenyl)piperidin-1-sulfonylmethyl]-*N*-hydroxy-3-methylbutyramid;
1-[4-(2-Methoxyphenyl)piperidin-1-sulfonylmethyl]cyclobutancarbonsäure-hydroxyamid;
1-[4-(2-Methylphenyl)piperidin-1-sulfonylmethyl]cyclobutancarbonsäure-hydroxyamid;
und ihre Salze, Solvate, Hydrate, Tautomere, Isomere oder *N*-Oxide handelt.

19. Pharmazeutische Zusammensetzung, die eine Verbindung nach Anspruch 1 zusammen mit einem oder mehreren pharmazeutisch unbedenklichen Trägern, Grundstoffen oder Verdünnungsmitteln enthält.

## Revendications

1. Composé de formule (1) : 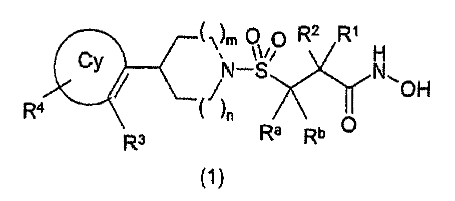 dans laquelle :
Cy est un groupement aryle ou hétéroaryle ;
m est zéro ou le nombre entier 1, 2 ou 3 ;
n est zéro ou le nombre entier 1, 2 ou 3 ; où la somme de m et de n est zéro ou le nombre entier 1, 2 ou 3 ;
R¹ est un groupement choisi parmi alkyl(C₁₋₆), aryle, hétéroaryle, hétérocycloalkyle, cycloalkyl(C₃₋₆), -alkyl(C₁₋₆)aryle, -alkyl(C₁₋₆)hétéroaryle, -alkyl(C₁₋₆)hétérocycloalkyle ou -alkyl(C₁₋₆)cycloalkyl(C₃₋₆), dans lequel chaque groupement aryle ou hétéroaryle, présent sous forme de, ou faisant partie du, groupement R¹, peut éventuellement être substitué par 1, 2 ou 3 substituants choisis parmi le groupement R⁷,où chaque R⁷ peut être identique ou différent, et est un atome ou un groupement choisi parmi F, Cl, Br, alkyl(C₁₋₆), halogénoalkyl(C₁₋₆), alcoxy(C₁₋₆), halogénoalcoxy(C₁₋₆), -CN, -CO2R^{7a}, -CON(R^{7a})₂ ou -COR^{7a} ; et dans lequel chaque groupement alkyle, hétérocycloalkyle ou cycloalkyle, présent sous forme de, ou faisant partie du, groupement R¹, peut éventuellement être substitué par 1, 2 ou 3 substituants choisis parmi le groupement R⁸, où chaque R⁸ peut être identique ou différent, et est un atome ou un groupement choisi parmi F, alkyl(C₁₋₆), halogénoalkyl(C₁₋₆), alcoxy (C₁₋₆), halogénoalcoxy(C₁₋₆), =O, =NOR¹⁰, -CO₂R^{8a}, -CON(R^{8a})₂ ou -COR^{8a} ;
R^{7a}, qui peut être identique ou différent, est chacun un atome d'hydrogène, ou un groupement alkyl(C₁₋₆) ou halogénoalkyl(C₁₋₆) ;
R^{8a}, qui peut être identique ou différent, est chacun un atome d'hydrogène, ou un groupement alkyl(C₁₋₆) ou halogénoalkyl(C₁₋₆) ;
R¹⁰ est un atome d'hydrogène ou un groupement alkyl(C₁₋₃) ;
R² est un atome d'hydrogène ou un groupement alkyl(C₁₋₃) ;
ou R¹ et R², conjointement avec l'atome de carbone auquel ils sont fixés, forment un groupement cycloalkyl(C₃₋₆) ou hétérocycloalkyle éventuellement substitué par 1, 2 ou 3 substituants choisis parmi le groupement R⁹, où chaque R⁹ peut être identique ou différent, et est un atome ou un groupement choisi parmi F, alkyl(C₁₋₆), halogénoalkyl (C₁₋₆), alcoxy (C₁₋₆), halogénoalcoxy(C₁₋₆), =O, =NOR¹⁰, -CO₂R^{8a}, -CON(R^{8a})₂ ou -COR^{8a} ;
R³ est un atome ou un groupement choisi parmi F, Cl, Br, alkyl(C₁₋₃), halogénoalkyl (C₁₋₃) alcoxy (C₁₋₃), halogénoalcoxy(C₁₋₃) ou -CN ;
R⁴ est un atome d'hydrogène, de F, de Cl ou de Br ou un groupement alkyl(C₁₋₃), halogénoalkyl(C₁₋₃), alcoxy(C₁₋₃), halogénoalcoxy(C₁₋₃), -CN, -SO₂R⁵, -SO₂N(R⁶)2, -CON(R⁶)₂, -N(R⁶)₂, -NHSO₂R⁵ ou -NHCOR⁵ ;
R⁵ est un groupement alkyl(C₁₋₃) ;
R⁶, qui peut être identique ou différent, est chacun un atome d'hydrogène ou un groupement alkyl(C₁₋₃) ; et
R^{a} et R^{b}, qui peuvent être identiques ou différents, sont chacun un atome ou un groupement choisi parmi hydrogène ou alkyl(C₁₋₃), ou R^{a} et R^{b} peuvent être liés pour former un groupement cycloalkyl(C₃₋₆) ou hétérocycloalkyle tel que défini pour R¹ et R² ;
et les sels, les solvats, les hydrates, les tautomères, les isomères ou les N-oxydes de celui-ci.

2. Composé selon la revendication 1, répondant à la formule (2) : 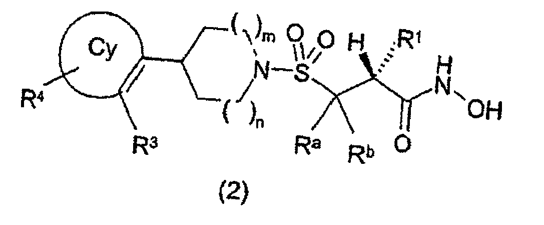 dans laquelle m, n, Cy, R^{a}, R^{b}, R¹, R³ et R⁴ sont tels que définis selon la revendication 1 ;
et les sels, les solvats, les hydrates, les tautomères, les isomères ou les N-oxydes de celui-ci.

3. Composé selon la revendication 1, répondant à la formule (3) : 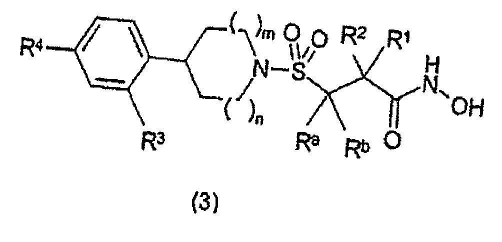 dans laquelle m, n, R^{a}, R^{b}, R¹, R², R³ et R⁴ sont tels que définis selon la revendication 1 ;
et les sels, les solvats, les hydrates, les tautomères, les isomères ou les N-oxydes de celui-ci.

4. Composé selon la revendication 3, répondant à la formule (4) : 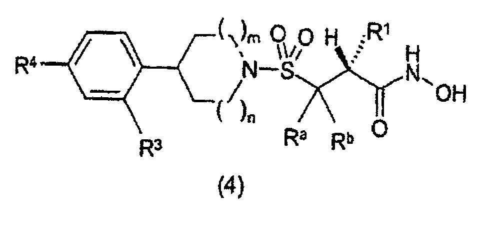 dans laquelle m, n, R^{a}, R^{b} R¹, R³ et R⁴ sont tels que définis selon la revendication 1 ;
et les sels, les solvats, les hydrates, les tautomères, les isomères ou les N-oxydes de celui-ci.

5. Composé selon la revendication 1 ou la revendication 2, dans lequel Cy est un groupement phényle.

6. Composé selon l'une quelconque des revendications précédentes, dans lequel R^{a} et R^{b} sont chacun un atome d'hydrogène.

7. Composé selon l'une quelconque des revendications précédentes, dans lequel m est le nombre entier 1 et n est zéro ou le nombre entier 1.

8. Composé selon l'une quelconque des revendications précédentes, dans lequel n est le nombre entier 1.

9. Composé selon l'une quelconque des revendications précédentes, dans lequel R¹ est un groupement choisi parmi alkyl(C₁₋₆), phényle, hétéroaryle, hétérocycloalkyle, cycloalkyl(C₃₋₆), -(CH₂)₁₋₂phényle, -(CH₂)₁₋₂hétéroaryle, -(CH₂)₁₋₂hétérocycloalkyle ou -(CH₂)₁₋₂cycloalkyl(C₃₋₆), dans lequel chaque groupement phényle ou hétéroaryle, présent sous forme de, ou faisant partie du, groupement R¹, peut éventuellement être substitué par 1, 2 ou 3 substituants choisis parmi le groupement R⁷, tel que défini selon la revendication 1 ; et dans lequel chaque groupement alkyle, hétérocycloalkyle ou cycloalkyle, présent sous forme de, ou faisant partie du, groupement R¹, peut éventuellement être substitué par 1, 2 ou 3 substituants choisis parmi le groupement R⁸, tel que défini selon la revendication 1.

10. Composé selon l'une quelconque des revendications précédentes, dans lequel R¹ est un groupement choisi parmi alkyl(C₁₋₆), phényle, hétérocycloalkyle, cycloalkyl(C₃₋₆) ou -(CH₂)₁₋₂phényle éventuellement substitué.

11. Composé selon l'une quelconque des revendications 1, 3 ou 5 à 8, dans lequel R¹ et R², conjointement avec l'atome de carbone auquel ils sont fixés, forment un groupement cycloalkyl(C₃₋₆) éventuellement substitué par 1, 2 ou 3 substituants choisis parmi le groupement R⁹, tel que défini selon la revendication 1.

12. Composé selon la revendication 11, dans lequel R¹ et R², conjointement avec l'atome de carbone auquel ils sont fixés, forment un groupement cyclobutyle.

13. Composé selon l'une quelconque des revendications précédentes, dans lequel R³ est un atome ou un groupement choisi parmi F, Cl, méthyle, éthyle, isopropyle, -CF₃, -CF₂H, méthoxy, éthoxy, -OCF₃, -OCF₂H ou -CN.

14. Composé selon l'une quelconque des revendications précédentes, dans lequel R⁴ est un atome ou un groupement choisi parmi un atome d'hydrogène, de F ou de Cl, ou un groupement méthyle, -CF₃, méthoxy, ou -OCF₂H.

15. Composé selon l'une quelconque des revendications précédentes, dans lequel R³ est un atome ou un groupement choisi parmi F, Cl, alkyl(C₁₋₃) ou alcoxy(C₁₋3)

16. Composé selon la revendication 15, dans lequel R³ est un groupement alkyl(C₁₋₃) ou alcoxy(C₁₋₃).

17. Composé selon la revendication 15 ou la revendication 16, dans lequel R³ est un groupement méthyle ou méthoxy.

18. Composé selon la revendication 1, qui est :
le 2-[4-(2-méthoxyphényl)pipéridine-1-sulfonylméthyl]-N-hydroxy-3-méthylbutyramide ;
le 2-[4-(2-méthyl-4-fluorophényl)pipéridine-1-sulfonylméthyl]-N-hydroxy-3-méthylbutyramide ;
le 2-benzyl-N-hydroxy-3-[4-(2-méthoxyphényl)pipéridine-1-sulfonyl]propionamide ;
le 2-benzyl-N-hydroxy-3-[4-(2-méthylphényl)pipéridine-1-sulfonyl]propionamide ;
le N-hydroxy-3-[4-(2-méthoxyphényl)pipéridine-1-sulfonyl]-2-phénylpropionamide ;
le 2(R)-[4-(2-méthoxyphényl)pipéridine-1-sulfonylméthyl]-N-hydroxy-3-méthylbutyramide ;
le 2(R)-[4-(2-méthylphényl)pipéridine-1-sulfonylméthyl]-N-hydroxy-3-méthylbutyramide ;
l'acide 1-[4-(2-méthoxyphényl)pipéridine-1-sulfonylméthyl]cyclobutanecarboxylique hydroxyamide ;
l'acide 1-[4-(2-méthylphényl)pipéridine-1-sulfonylméthyl]cyclobutanecarboxylique hydroxyamide ;
et les sels, les solvats, les hydrates, les tautomères, les isomères ou les N-oxydes de celui-ci.

19. Composition pharmaceutique comprenant un composé selon la revendication 1, conjointement avec un ou plusieurs véhicules, excipients ou diluants pharmaceutiquement acceptables.
